(19) Europäisches Patentamt
European Patent Office
Office européen des brevets

(11) Publication number: **0 004 835**
**B1**

(12) **EUROPEAN PATENT SPECIFICATION**

(45) Date of publication of patent specification: **14.10.81**

(21) Application number: **79850021.1**

(22) Date of filing: **03.04.79**

(51) Int. Cl.[3]: **C 07 C 93/14,**
A 61 K 31/135,
C 07 C 91/34, C 07 C 93/26

(54) **Ethanolamine compounds, processes for their preparation, pharmaceutical preparations containing them and their medicinal use.**

(30) Priority: **10.04.78 GB 1403378**

(43) Date of publication of application:
**17.10.79 Bulletin 79/21**

(45) Publication of the grant of the European patent:
**14.10.81 Bulletin 81/41**

(84) Designated Contracting States:
**BE CH DE FR GB IT LU NL SE**

(56) References cited:
FR - A - 2 005 687
FR - M - 7 198
DE - A - 2 008 654
DE - A - 2 711 981

**Chemical Abstracts, Volume 80, 1974 (COLUMBUS, OHIO, USA)**
**J. VAN DIJK et al. "Synthesis of β-phenylethylamine derivatives. X.N.-(Hydroxy- and methoxyaralkyl) derivatives.**
**page 366, abstract Nr. 95398z**

(73) Proprietor: **Aktiebolaget DRACO**
**Fack**
**S-221 01 Lund 1 (SE)**

(72) Inventor: **Olsson, Otto Agne Torsten**
**Hjortgatan 3**
**S-223 50 Lund (SE)**
Inventor: **Persson, Nils Henry Alfons**
**Langgatan 3**
**S-240 10 Dalby (SE)**
Inventor: **Svensson, Leif Ake**
**Stangby 17**
**S-225 90 Lund (SE)**
Inventor: **Waldeck, Carl Bertil**
**Sofielundsvägen 34**
**S-240 17 S. Sandby (SE)**
Inventor: **Wetterlin, Kjell Ingvar Leopold**
**Västervang 19**
**S-240 17 S.Sandby (SE)**

(74) Representative: **Wurm, Bengt R. et al,**
**S-151 85 Södertälje (SE)**

**EP 0 004 835 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Ethanolamine compounds, processes for their preparation, pharmaceutical preparations containing them and their medicinal use

*Field of the invention*

The present invention relates to new compounds which are effective in the treatment of reversible obstructive lung conditions of various genesis particularly asthmatic conditions, but which exhibit reduced heart stimulating activity and reduced tremorogenic activity, the preparation of such compounds, pharmaceutical compositions containing them, and the use for therapeutic purposes of such compounds.

*Background of the invention*

Sympathomimetic amines are generally used as bronchodilating agents in the treatment of reversible obstructive lung conditions such as asthma. J. Van Dijk et al disclose in Recl. Trav. Chim. Pays-Bas 1973, Volume 92, No. 12, pages 1281—1297, β-phenylethylamine derivatives having uterus relaxing activity. Sympathomimetic amines such as adrenaline and isoprenaline exhibit, however, the undesired effect of being strongly heart stimulating. Subsequently available bronchospasmolytic agents such as terbutaline are bronchoselective, which means that they exhibit much less of heart stimulating effect in relation to their bronchodilating effect, but they still often have the undesired side effect of being tremorogenic, which naturally can be very disturbing to the patient. The tremorogenic effects of β-stimulating agents are discussed e.g. by Marsden et al, Clin Sci 1967, *33*, 53—65, and by Marsden and Meadows, J. Physiol 1970, *207*, 429—448.

Thus, there is a need for bronchospasmolytic agents having a pronounced bronchodilating effect particularly at oral administration while showing a low heart-stimulating effect and a low tremorogenic effect.

*Outline of the invention*

The present invention provides new compounds which have a pronounced bronchodilating activity while they exhibit a low heart-stimulating effect and a low tremorogenic effect. The invention also relates to methods for preparation of the compounds, pharmaceutical preparations containing the compounds as active ingredients and to the use of the compounds for therapeutic purposes, in particular the use for producing bronchodilation in mammals including humans. The compounds of the invention have also proved to have α-receptor blocking effect, and the invention also relates to their use as α-receptor blocking agents in mammals including humans. Drugs combining α-adrenoceptor blocking properties and β-adrenoceptor stimulating properties may be beneficial in the treatment of specific diseases such as asthma (Aggerbeck et al., Br. J. Pharma (1979) 65 155—159; Nousiainen et al., Pharmacology (1977) 15 469—477. A still further aspect of the invention is the use of the compounds for producing relaxation of the human uterus.

*Detailed description of the invention*

The invention relates to the new compounds of the formula

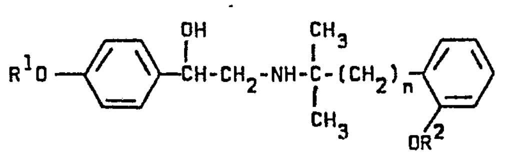

I

and therapeutically acceptable salts thereof, in which formula n is 1, 2 or 3; $R^1$ is selected from the group consisting of H, aliphatic acyl groups containing 2 to 5 carbon atoms, and benzoyl of the formula

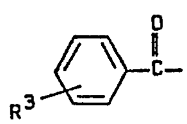

wherein $R^3$ is selected from the group consisting of hydrogen and methyl; and wherein $R^2$ is selected from the group consisting of H, aliphatic alkyl groups containing 1—4 carbon atoms, benzyl, aliphatic acyl groups containing 2 to 5 carbon atoms, and benzoyl of the formula

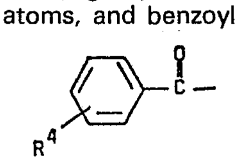

wherein $R^4$ is selected from the group consisting of hydrogen and methyl.

The radicals $R^3$ and $R^4$ can be situated in any of the positions 2, 3 and 4 on the phenyl ring.

Illustrative examples of the radicals $R^1$ and $R^2$ are:

Hydrogen, acyl groups ($R^1$ and $R^2$), $CH_3CO—$, $CH_3CH_2CO—$, $CH_3CH_2CH_2CO—$, $HC(CH_3)_2CO—$,

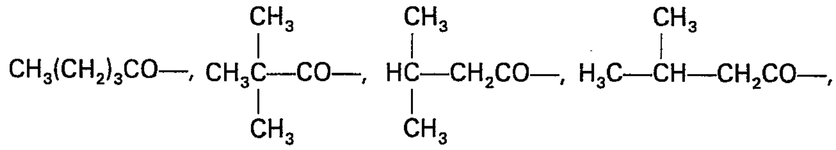

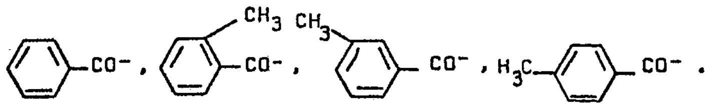

Alkyl groups ($R^2$ only):
$—CH_3$, $—C_2H_5$, $n\text{-}C_3H_7$, $i\text{-}C_3H_7$, $n\text{-}C_4H_7$, $sec\text{-}C_4H_7$, $iso\text{-}C_4H_7$.

Illustrative examples of compounds of the invention are:

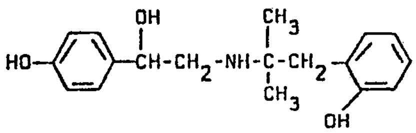
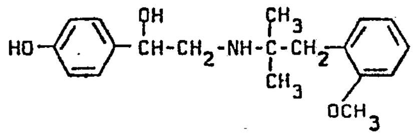

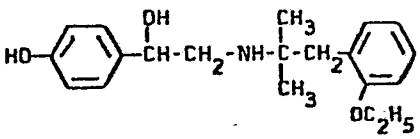
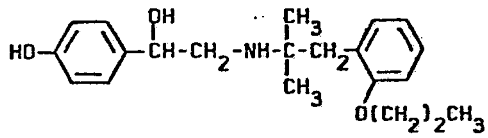

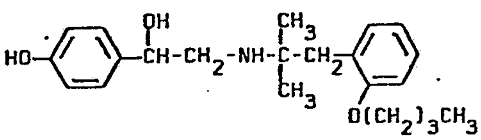
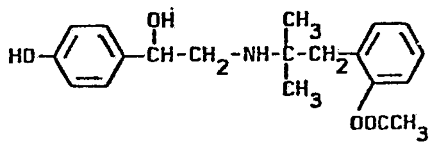

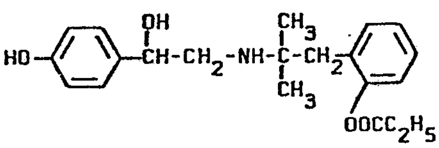
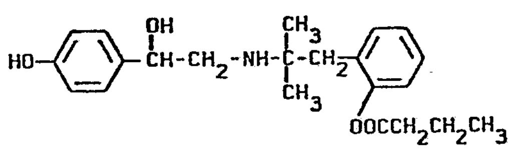

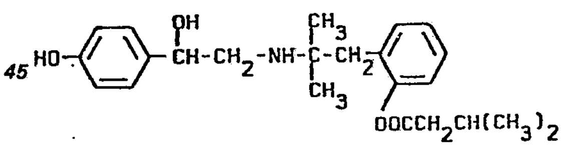
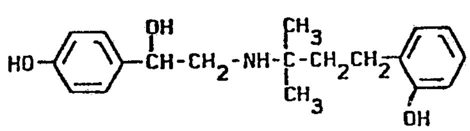

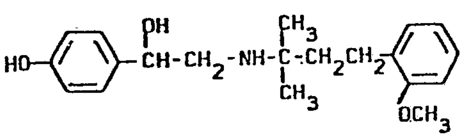
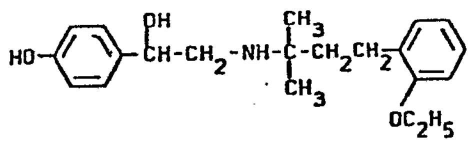

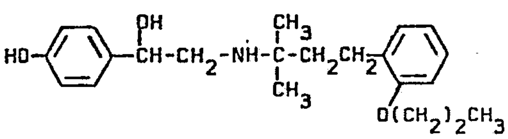
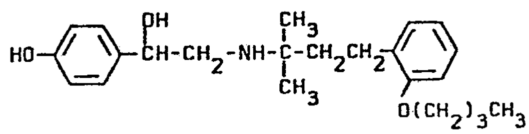

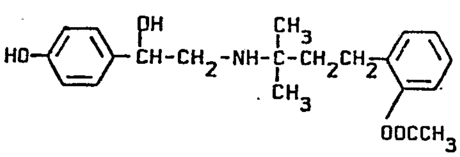
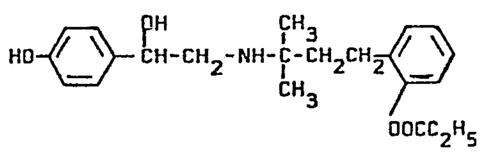

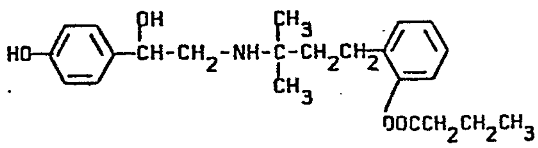

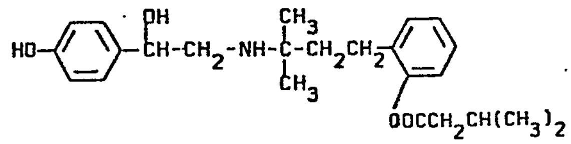

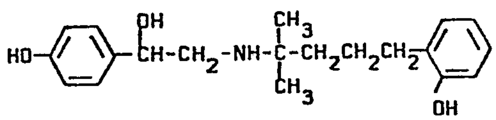

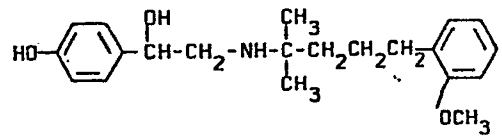

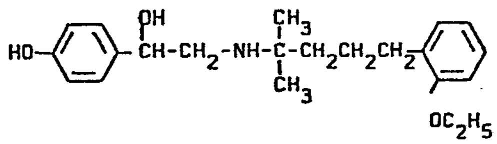

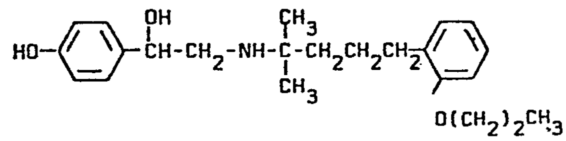

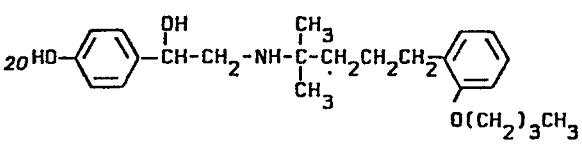

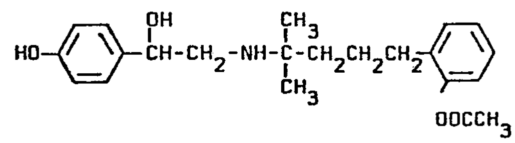

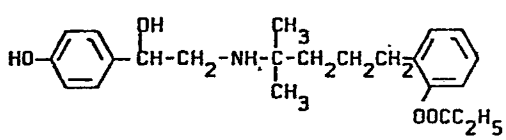

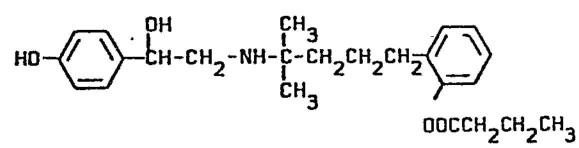

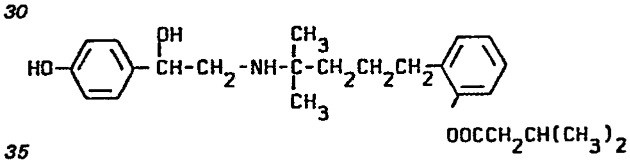

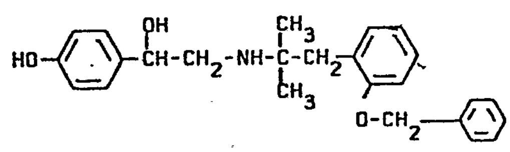

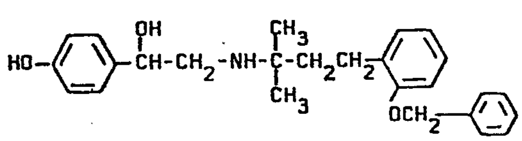

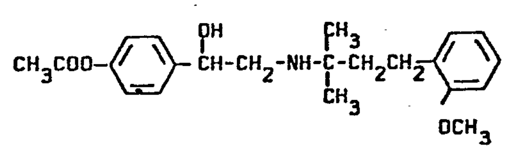

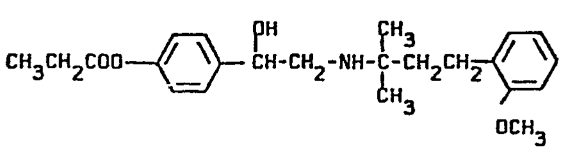

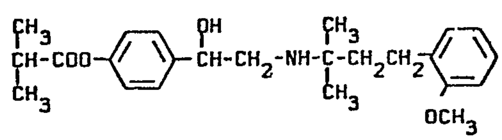

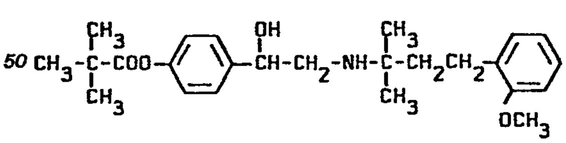

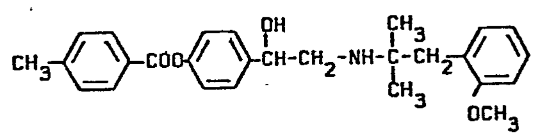

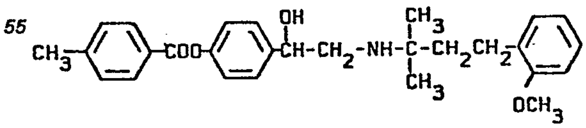

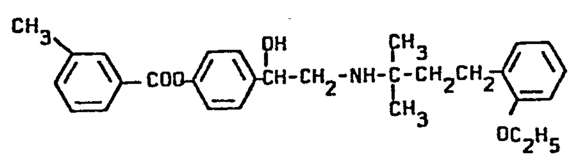

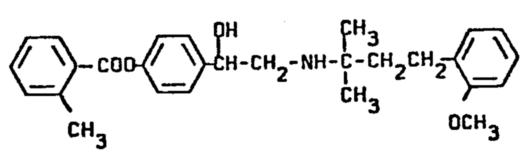

Examples of preferred groups of compounds within the formula are given below:

1. Compounds wherein $R^1$ is hydrogen.
2. Compounds wherein $R^2$ is an aliphatic acyl group containing 2—5 carbon atoms.
3. Compounds wherein n is 2 (two).
4. Compounds wherein n is 1 (one).
5. Compounds wherein $R^2$ is an alkyl group containing 1—4 carbon atoms.
6. Compounds wherein n is 2 (two), $R^1$ is hydrogen, and $R^2$ is an alkyl group containing 1—4 carbon atoms.
7. Compounds wherein n is 2, $R^1$ is an aliphatic acyl group containing 2—5 carbon atoms or benzoyl of the formula

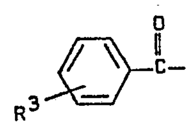

wherein $R^3$ is H or $CH_3$, and $R^2$ is an alkyl group containing 1—4 carbon atoms.

8. Compounds wherein n is 1 (one), $R^1$ is hydrogen, and $R^2$ is an alkyl group containing 1—4 carbon atoms.
9. Compounds wherein n is 1, $R^1$ is an aliphatic acyl group containing 2—5 carbon atoms or benzoyl of the formula

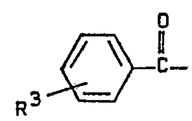

wherein $R^3$ is H or $CH_3$, and $R^2$ is an alkyl group containing 1—4 carbon atoms.

The preferred compound of the invention is

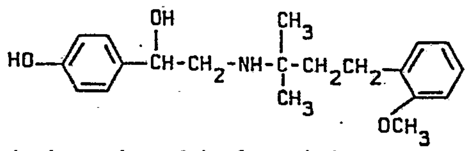

As the compounds of the invention of the formula I possess at least one asymmetric carbon atom, the invention includes all the possible optically active forms and racemic mixtures of the compounds. The racemic mixture may be resolved by conventional methods, for example by salt formation with an optically active acid, followed by fractional crystallization.

In clinical practice the compounds will normally be administered orally, by injection or by inhalation in the form of a pharmaceutical preparation comprising the active ingredient in the form of the original compound or optionally in the form of a pharmaceutically acceptable salt thereof, in association with a pharmaceutically acceptable carrier which may be a solid, semisolid or liquid diluent or an ingestible capsule, and such preparations comprise a further aspect of the invention. The compounds may also be used without carrier material. As examples of pharmaceutical preparations may be mentioned tablets, drops, aerosol for inhalation, etc. Usually the active substance will comprise between 0.05 and 99, or between 0.1 and 99% by weight of the preparation, for example between 0.5 and 20% for preparation intended for injection and between 0.1 and 50% for preparations intended for oral administration.

The new compounds according to the invention may be administered in the form of salts with physiologically acceptable acids. Suitable acids which may be used are, for example, hydrochloric, hydrobromic, sulphuric, fumeric, citric, tartaric, maleic or succinic acid.

The invention further provides pharmaceutical compositions comprising as active ingredient at least one of the compounds according to the invention in association with a pharmaceutical carrier. Such compositions may be designed for oral, bronchial, rectal or parenteral administration.

To produce pharmaceutical preparations in the form of dosage units for oral application containing a compound of the invention in the form of the free base, or a pharmaceutically acceptable salt thereof, the active ingredient may be mixed with a solid, pulverized carrier, for example, lactose, saccharose, sorbitol, mannitol, a starch such as potato starch, corn starch, maize starch or amylopectin, a cellulose derivative or gelatin, and also may include lubricants such as magnesium or calcium stearate or a Carbowax® or other polyethylene glycol waxes and compressed to form tablets or centers for dragees. If dragees are required, the centers may be coated, for example, with concentrated sugar solutions which may contain gum arabic, talc and/or titanium dioxide, or alternatively with a lacquer dissolved in easily volatile organic solvents or mixtures of organic solvents. Dyestuffs can be added to these coatings. For the preparation of soft gelatin capsules (pearl-shaped closed capsules) consisting of gelatin and, for example, glycerol, or similar closed capsules, the active substance may be admixed with a Carbowax®. Hard gelatin capsules may contain granulates of the active substance with solid, pulverized carriers such as lactose, saccharose, sorbitol, mannitol, starches (for example potato starch,

corn starch, or amyllopectin), cellulose derivatives or gelatin, and may also include magnesium stearate or stearic acid. Dosage units for rectal application may be in the form of suppositories comprising the active substance in admixture with a neutral fatty base, or gelatin rectal capsules comprising the active substance in admixture with a Carbowax® or other polyethylene glycol waxes. Each dosage unit preferably contains 0.5 to 50 mg active ingredient.

Liquid preparations for oral application may be in the form of syrups, suspensions or emulsions, for example containing from about 0.1% to 20% by weight of active substance and also, if desired, such adjuvants as stabilizing agents, suspending agents, dispersing agents, flavouring agents and/or sweetening agents.

Liquid preparations for rectal administration may be in the form of aqueous solutions containing from about 0.1% to 2% by weight of active substance and also, if desired, stabilizing agents and/or buffer substances.

For parenteral application by injection the carrier may be a sterile, parenterally acceptable liquid, e.g. pyrogen-free water or an aqueous solution of polyvinylpyrrolidone, or a parenterally acceptable oil, e.g., arachis oil and optionally stabilizing agents and/or buffer substances. Dosage units of the solution may advantageously be enclosed in ampoules, each dosage unit preferably containing from 0.05 to 5 mg of active ingredient.

For administration to the bronchia, the compositions are advantageously in the form of a spray solution or spray suspension. The solution or suspension advantageously contains from 0.1 to 10% by weight of the active ingredient.

The dosage at which the active ingredients are administered may vary within a wide range and will depend on various factors such as for example the individual requirements of each patient. A suitable oral dosage range may be from 0.5 to 10 mg per day.

At treatment with dose aerosol, a suitable dosage unit may contain from 0.05 to 0.5 mg of the active ingredient. One or two such dosage units may be administered at each treatment.

The pharmaceutical compositions containing the active ingredients may suitably be formulated so that they provide doses within these ranges either as single dosage units or as multiple dosage units.

The compounds of the formula I wherein $R^1$ and $R^2$ are aliphatic acyl groups or unsubstituted or substituted benzoyl show as rapid onset as the corresponding compounds wherein $R^1$ and/or $R^2$ are hydrogen but longer duration of the pharmacological effect. This is probably due to a better absorption followed by hydrolysis of the ester linkage in the organism. The compounds wherein $R^1$ and $R^2$ are aliphatic acyl groups or unsubstituted or substituted benzoyl thus act as prodrugs for the corresponding compounds wherein $R^1$ and/or $R^2$ are hydrogen.

The compounds of the invention are prepared by known methods such as

A. reacting a compound of the formula

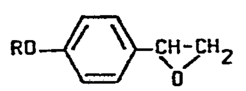

III

wherein R is hydrogen; an aliphatic acyl group containing 2 to 5 carbon atoms, benzoyl of the formula

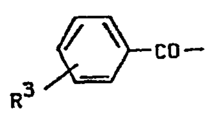

wherein $R^3$ is hydrogen or methyl; or a hydroxy-protecting group such as an alkyl group containing 1—5 carbon atoms or a mono- or bicyclic aralkyl group of not more than 11 carbon atoms such as benzyl or naphtylmethyl; with a compound of the formula

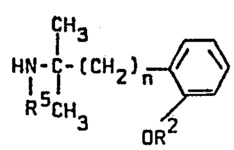

IV

wherein n is 1, 2 or 3; $R^2$ is selected from the group consisting of hydrogen; aliphatic alkyl groups containing 1—4 carbon atoms; benzyl; aliphatic acyl groups containing 2 to 5 carbon atoms, and benzoyl of the formula

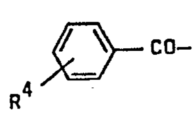

wherein $R^4$ is hydrogen or methyl; and wherein $R^5$ is hydrogen or an N-protecting group such as benzyl, to the formation of a compound of the formula

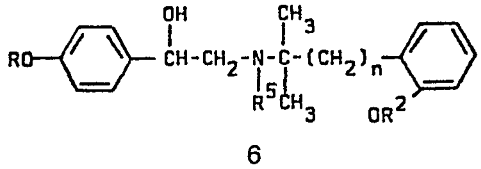

V

whereafter, if necessary, protecting groups R and $R^5$ are removed;

B. reducing a compound of the formula

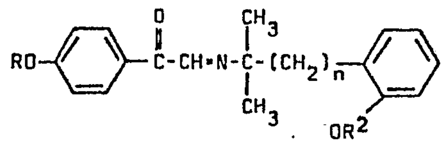

VI

wherein n, R and $R^2$ have the meanings indicated under A above, to the formation of a compound of the formula

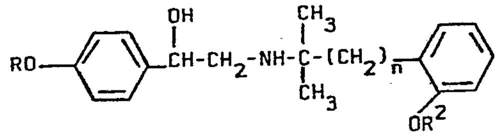

V

whereafter, if necessary, the protecting group R is removed; whereafter, if desired, the compound of the formula I thus obtained is converted into a pharmaceutically acceptable salt and/or resolved into its optical isomers.

The starting materials utilized in the reactions A and B above are known compounds which can be prepared in known manner.

The following examples illustrate the invention.

Example 1

*Method A: Preparation of 1-(4-hydroxyphenyl)-2-[1,1-dimethyl-3-(2-methoxyphenyl)propylamino]-ethanol hydrochloride*

a) Preparation of 1-(4-benzyloxyphenyl)-2-[1,1-dimethyl-3-(2-methoxyphenyl)propylamino]-ethanol sulfate

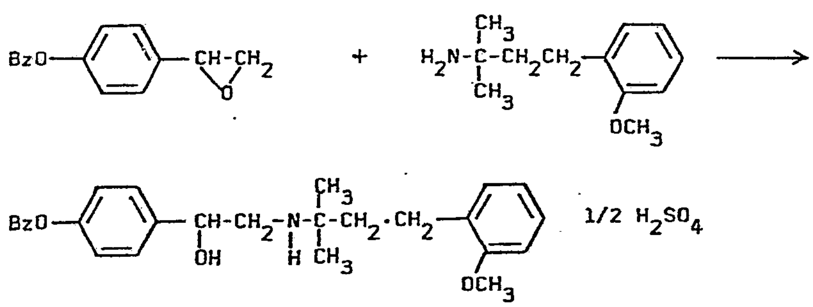

A solution of 3.5 g (0.018 mole) 1,1-dimethyl-3-(2-methoxyphenyl) propylamine, and 3.8 g (0.017 mole) 4-benzyloxystyrene oxide in 150 ml of n-propanol was boiled under reflux for two days. The reaction mixture was evaporated to dryness *in vacuo,* and the residue dissolved in EtOH. After neutralization with conc $H_2SO_4$ in EtOH a crystalline precipitate was formed. After two recrystallizations from i-propanol, and a final one from EtOH, a yield of 0.9 g of the title compound was obtained.

Sulfate $_{calc}$: 100%
Sulfate $_{found}$: 101%
NMR data:
$CDCl_3$ $\delta$ (ppm): 1.15 (6H,s) 1.60 (2H,m) 2.60 (2H+2H,m) 3.75 (3H,s) 4.55 (1H,q) 5.07 (2H,s) 7,10 (4H+4H+5H,m)

This compound was hydrogenated as described in b) below.

b) Preparation of 1-(4-hydroxyphenyl)-2-[1,1-dimethyl-3-(2-methoxyphenyl)propylamino]-ethanol hydrochloride

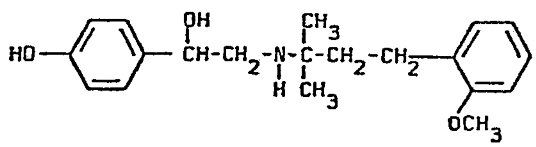

; HCl

A solution of 3.1 g 1-(4-benzyloxyphenyl)-2-[1,1-dimethyl-3-(2-methoxyphenyl) propylamino] ethanol in 40 ml i-propanol was hydrogenated in the presence of 0.05 g 10% Pd/C at atmospheric pressure and room temperature. When calculated amount $H_2$ has been consumed, 1 g benzyl chloride was added together with some fresh catalyst. Hydrogenation was continued until hydrogen uptake ceased. The catalyst was filtered off, and the filtrate concentrated *in vacuo*. The hydrochloride of the title compound crystallized upon addition of $Et_2O$.

Yield: 2.5 g $Cl^-_{calc}$: 9.7%
$Cl^-_{found}$: 9.7%

NMR data:

δ (ppm): 1.55 (6H,s) 1.90 (2H,m) 2.77 (2H,m) 3.20 (2H,m) 3.85 (3H,s) 4.85 (DOH) 5.05 (1H,m) 7.32 (8H,m)

MS: (M—18) m/e = 311
di-TMS-derivate $M^+$ m/e = 473 (M—15) m/e = 458

Example 2

*Method A: Preparation of 1-(4-hydroxyphenyl)-2-[1,1-dimethyl)-2-(2-methoxyphenyl)ethylamino]-ethanol sulfate*

a) Preparation of 1-(4-Benzyloxyphenyl)-2-[1,1-dimethyl-2-(2-methoxyphenyl)ethylaminoethanol sulfate

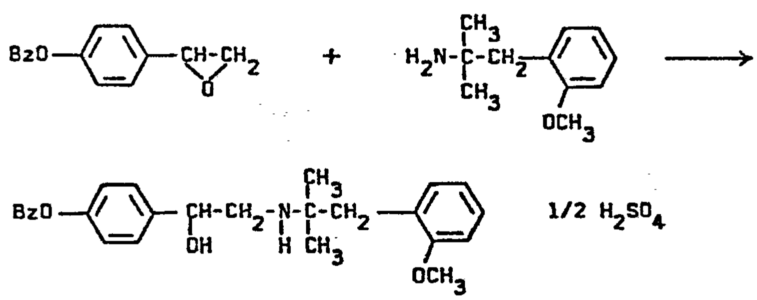

A solution of 7.2 g (0.04 mole) 1,1-dimethyl-2-(2-methoxyphenyl) ethylamine, and 9.1 g (0.04 mole) 4-benzyloxystyreneoxide in 120 ml EtOH was refluxed for 20 hours. After evaporation to dryness, the residue was dissolved in $Et_2O$, and neutralized with 2N $H_2SO_4$. The precipitate formed was filtered off, and washed with $H_2O$ and $Et_2O$. Recrystallized from EtOH.

Yield: 4.4 g. The product obtained was directly utilized in step b).

b) Preparation of 1-(4-Hydroxyphenyl)-2-[1,1-dimethyl-2-(2-methoxyphenyl)ethylamino]ethanol sulfate

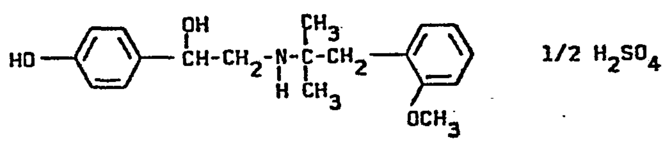

A solution of 2 g 1-(4-benzyloxyphenyl)-2-[1,1-dimethyl-2-(2-methoxyphenyl)ethylamino]-ethanol sulfate in 50 ml of MeOH was hydrogenated in the presence of 0.5 g 10% Pd/C at room temperature and atmospheric pressure. When calculated amount hydrogen had been consumed, the catalyst was filtered off, and the filtrate evaporated. The residue was recrystallized from EtOH.

Yield: 1.5 g $SO_4^{2-}$ 100.5%

NMR data:

($DMSO-d_6$) δ (ppm): 0.65 (6H,s) 1.92 (DMSO) 2.42 (4H,m) 3.10 (3H,s) 4.25 (1H,m) 6.50 (8H,m)

MS

TMS der: $M^+$ m/e = 460 (M—15) m/e = 445

Example 3

*Method B: Preparation of 1-(4-hydroxyphenyl)-2-[1,1-dimethyl-3-(2-methoxyphenyl)propylamino]-ethanol*

a) Preparation of 1-(4-benzyloxyphenyl)-2-[1,1-dimethyl-3-(2-methoxyphenyl)propylamino]-ethanol

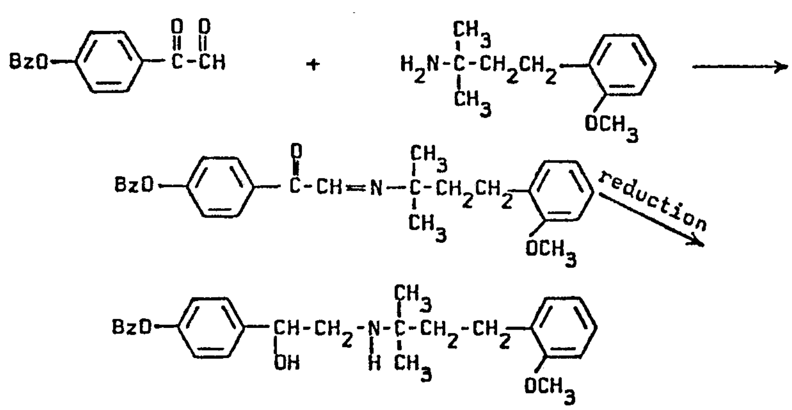

A solution of 3.0 g (0.0155 mole) 1,1-dimethyl-3-(2-methoxyphenyl)propylamine, and 4.0 g (0.0155 mole) 4-benzyloxyphenyl glyoxal in 40.0 ml EtOH is boiled under reflux for 1 hr, and then stirred at room temperature for 20 hours. The intermediate iminoketone is thereafter directly reduced by addition of 1.5 g $NaBH_4$ and the solution is stirred for another hour. After evaporation of the reaction mixture to dryness *in vacuo,* the residue is partitioned between $Et_2O$ and $H_2O$. The ether phase was washed with $H_2O$, dried over $MgSO_4$, and evaporated.

The crystalline residue was recrystallized from EtOH.

Yield: 3.1 g

NMR data for the intermediate iminoketone:

($CDCL_3$) $\delta$ (ppm): 1.25 (6H,s) 1.82 (2H,m) 2.60 (2H,m) 3.75 (3H,s) 5.10 (2H,s) 7.18 (11H,m) 8.10 (2H,d) 8.35 (1H,s)

b) Preparation of 1-(4-hydroxyphenyl)-2-[1,1-dimethyl-3(2-methoxyphenyl)propylamino]-ethanol

The compound obtained in step a) was hydrogenated in the same manner as described in Example 1 b to give the title compound.

Example 4

*Method B: Preparation of 1-[4-Pivaloyloxyphenyl]-2-[1,1-dimethyl-3-(2-methoxyphenyl)propylamino] ethanol sulfate*

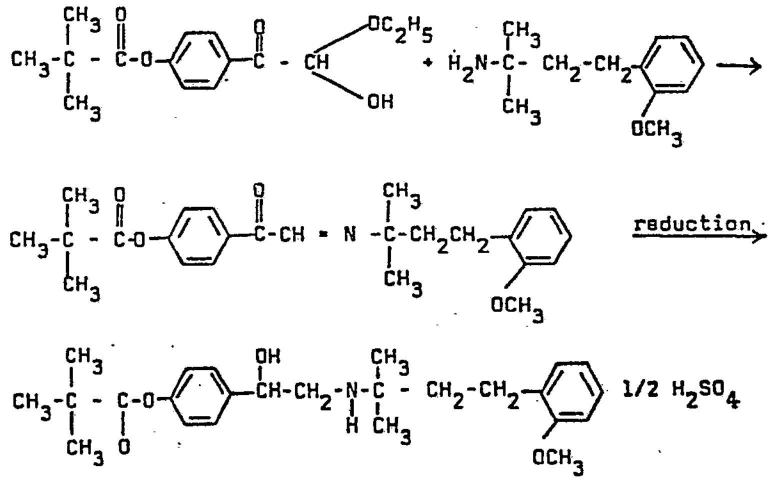


A solution of 2 g 1,1-dimethyl-3-[2-methoxyphenyl]-propylamine, and 2.9 g 4-pivaloyloxyphenyl glyoxal monoethyl acetal in 100 ml EtOH is refluxed for 2 hours, and then allowed to stand at room temperature for 20 hours.

To this solution $B_2H_6$, prepared from 23 ml of $BF_3$-etherate and 3.4 g $NaBH_4$ in 110 ml of diglyme, is introduced by means of a stream of $N_2$. When the reaction is completed water is carefully added, and the reaction mixture evaporated to dryness. The residue is extracted with $Et_2O$, which is then washed with $H_2O$, dried and evaporated. The residue was dissolved in EtOH, and neutralized with ethanolic sulfuric acid. The crystalline residue obtained after evaporation was recrystallized from EtOH.

Yield: 1.6 g 100% neutral sulfate

NMR: ($CDCl_3$, $CF_3COOH$) $\delta$ (ppm) 1.40 (9H,s) 1.52 (6H,s) 2.00 (2H,m) 2.90 (2H,m) 3.25 (2H,m) 3.90 (3H,s) 5.25 (1H,m) 7.18 (8H,m)

Example 5

*Method B: Preparation of 1-[4-(4-Methylbenzoyloxyphenyl]-2-[1,1-dimethyl-3-(2-methoxyphenyl)-propylamino] ethanol sulfate*

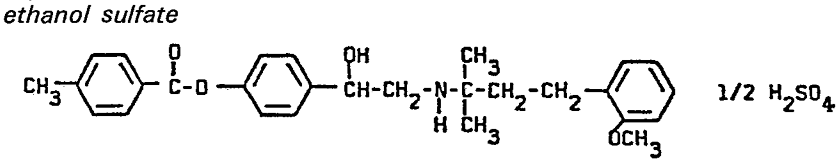


The title compound was prepared in the same manner as described in Example 4 for 1-[4-pivaloyloxyphenyl]-2-[1,1-dimethyl-3-(2-methoxyphenyl)propylamino] ethanol sulfate from 6.0 g 1,1-dimethyl-3-(2-methoxyphenyl)propylamine, and 9.8 g of 4-[4-methylbenzoyloxy]phenylglyoxal monoethyl acetal in 200 ml of EtOH. Recrystallization from i-PrOH.

Yield: 1.3 g 99.4% neutral sulfate

NMR data:

($CDCl_3$ + $CF_3COOH$) $\delta$ (ppm) 1.52 (6H,s) 2.02 (2H,m) 2.50 (3H,s) 2.75 (2H,m) 3.15 (2H,m) 3.85 (3H,s) 5.25 (1H,m) 7.20 (12H,m)

Example 6

*Preparation of 1-(4-hydroxyphenyl)-2-[1,1-dimethyl-3-(2-ethoxyphenyl)propylamino]-ethanol hydrochloride*

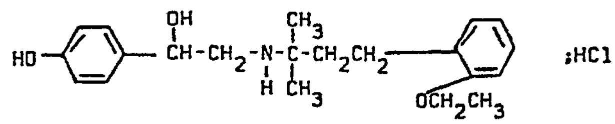

The title compound was prepared from 1-(4-benzyloxyphenyl)-2-[1,1-dimethyl-3-(2-ethoxyphenyl)propylamino]-ethanol in the same way as described in example 1b.

Yield: 15% Cl $_{calc}$: 9.0%

Cl $_{found}$: 9.0%

NMR (DMSO-$d_6$) $\delta$ (ppm) 1.1 (3H,t) 1.15 (6H,s) 1.7 (2H,m) 2.2 (DMSO) 2.9 (2H,m) 3.9 (2H,q) 4.8 (1H,m) 7.0 (8H,m)

Example 7

*Preparation of 1-(4-hydroxyphenyl)-2-[1,1-dimethyl-4-(2-methoxyphenyl)butylamino]-ethanolsulfate*

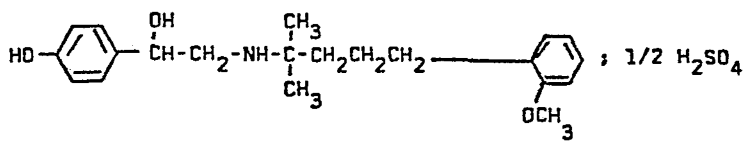

The title compound was prepared from 2.6 g 1-(4-benzyloxyphenyl)-2-[1,1-dimethyl-4-(2-methoxyphenyl)butylamino]-ethanolsulfate in the same way as described in example 1b.

Yield: 1.7 g Sulfate $_{calc}$: 100%

Sulfate $_{found}$: 100%

CIMS ($NH_3$) $MH^+$ 344

HPLC: 98.6%

Example 8

*Preparation of 1-(4-hydroxyphenyl)-2-[1,1-dimethyl-3-(2-hydroxyphenyl)propylamino]-ethanolsulfate*

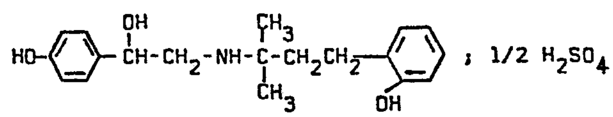

The title compound was prepared from 1.9 g 1-(4-benzoyloxyphenyl)-2-[1,1-dimethyl-3-(2-hydroxyphenyl)propylamino]ethanolsulfate in the same way as described in example 1b.

Yield: 1.4 g

HPLC analysis 96.8%

Sulfate $_{calc}$: 100%

Sulfate $_{found}$: 100%

CIMS ($NH_3$) $MH^+$: 316

NMR: ($CD_3OD$ + $CF_3COOH$) $\delta$ (ppm) 1.0 (6H,s) 1.5 (2H,m) 2.3 (2H,m) 2.8 (2H,m) 2.95 ($CD_3OD$) 4.5 (1H,m) 6.8 (8H,m)

*Preparation of starting materials*

a) Preparation of 1,1-dimethyl-3-[2-methoxyphenyl]-propylamine utilized in Methods A and B.

*Step 1:* 2-Methoxybenzalacetone

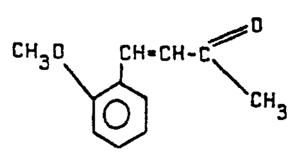

To a solution of 68 g (0.5 mole) o-methoxy benzaldehyde in 100 ml of acetone is added dropwise under stirring 12.5 ml of a 10% NaOH solution. The temperature is kept below 30°C, and the reaction mixture is stirred at room temperature for 3 hours, whereafter 2 N HCl is added, and the solution is boiled for 1 hour. The solution is then extracted with $Et_2O$, the ether phase washed with $H_2O$, dried, and evaporated to dryness *in vacuo*. The residue was distilled.

Yield: 32.4 g B.p.: 124—6°C/266 Pa (2 mm Hg)

NMR ($CDCl_3$) $\delta$ (ppm) 2.20 (3H,s) 3.90 (3H,s) 6.75 (1H,d) 7.15 (4H,m) 7.95 (1H,d)

*Step 2:* 4-[2-methoxyphenyl]-butan-2-on

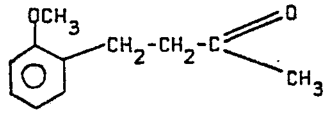

A solution of 32.4 g 2-methoxybenzalacetone in EtOH was hydrogenated in the presence of Raney-Ni at atmospheric pressure and room temperature. The catalyst was filtered off and the filtrate evaporated to yield the title compound as an oil.
Yield: 28.9 g
NMR: $(CDCl_3)$ $\delta$ (ppm) 2.10 (3H,s) 2.90 (4H,m) 3.90 (3H,s) 7.00 (4H,m)

*Step 3:* 1,1-Dimethyl-3-[2-methoxyphenyl]propanol

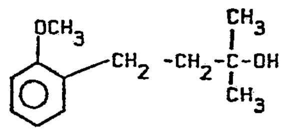

A solution of $CH_3Mg$ I (0.21 mole) in 50 ml dry $Et_2O$ was added dropwise with stirring and chilling to a solution of 28.9 g (0.16 mole) 4-[2-methoxyphenyl]-butan-2-on in 30 ml dry $Et_2O$. When the addition is completed the mixture is stirred for 2 hours at room temperature, whereafter $H_2O$ is added at 0°C. After extraction with $Et_2O$, the title compound is obtained after evaporation of the dried $Et_2O$ phase.
Yield: 28.6 g
NMR: $(CDCl_3)$ $\delta$ (ppm) 1.25 (6H,s) 1.75 (2H,m) 2.70 (2H,m) 3.90 (3H,s) 7.00 (4H,m)

*Step 4:* 1,1-Dimethyl-3-[2-methoxyphenyl]-propylamine

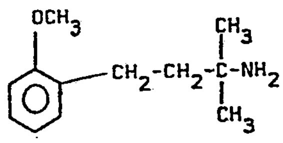

To a solution prepared by the addition with chilling of 22.4 ml conc $H_2SO_4$ in 20.6 ml HOAc to 8.6 g NaCN dissolved in 20.6 ml HOAc, was added dropwise 28.6 g 1,1-dimethyl-3-[2-methoxyphenyl]-propanol at a temperature of 40—50°C. The mixture was then stirred at 70°C for 0.5 hour. The reaction flask was stoppered and was left at room temperature for 2 hours, whereafter it was poured into 500 ml $H_2O$, and neutralized with $K_2CO_3$. A yield of 29.4 g of the intermediate formamide was isolated after extraction with $Et_2O$. This compound was, without further purification, hydrolyzed to the title compound by boiling it with 200 ml 5 N NaOH for 3 hours. The hydrolyzed mixture was steam distilled and the steam destillate extracted with $Et_2O$ which upon evaporation gave the title compound as an oil.
Yield: 14.4 g
NMR: $(CDCl_3)$ $\delta$ (ppm) 1.15 (6H,s) 1.52 (2H,m) 2.66 (2H,m) 3.85 (3H,s) 7.08 (4H,m)

b) Preparation of 4-benzyloxy styrene oxide utilized in Method A.

*Step 1:* 4-Benzyloxy-2'-bromoacetophenone

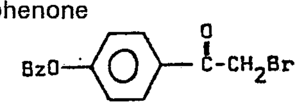

To a solution of 70.5 g (0.31 mole) 4-benzyloxyacetophenone in 500 ml dioxane was added dropwise under stirring a solution of 15.7 ml bromine in 400 ml dioxane. The mixture was stirred at room temperature for 2 hours, whereafter activated carbon was added, and the solution filtered. The filtrate was evaporated to dryness, and the solid residue was recrystallized from EtOH.
Yield: 73.2 g
NMR: $(CDCl_3)$ $\delta$ (ppm) 4.45 (2H,s) 5.15 (2H,s) 7.10 (1H,m) 7.50 (5H,s) 8.07 (1H,m)

*Step 2:* 4-Benzyloxy styrene oxide

A solution of 40 g 4-benzyloxy-2'-bromoacetophenone in 400 ml of dioxane was added dropwise to a solution of 5 g $NaBH_4$ in 40 ml $H_2O$ and 400 ml dioxane kept at 0°C. The mixture was stirred at room temperature for 20 hours, whereafter a solution of 9 g NaOH in 60 ml, $H_2O$ was added, and the solution warmed to reflux, and then cooled, and evaporated to dryness *in vacuo*. The residue was partitioned between $H_2O$ and $Et_2O$. The $Et_2O$-phase was dried, and evaporated to yield a crystalline residue: 27.3 g

NMR: ($CDCl_3$) of $\delta$ (ppm) 3.00 (2H,m) 3.85 (1H,m) 5.15 (2H,s) 7.15 (9H,m)

c) Preparation of 4-Benzyloxyphenyl glyoxal utilized in Method B.

To a solution of 32 g (0.14 mole) of p-benzyloxyacetophenone in 200 ml of dioxane was added 18 g (0.16 mole) $SeO_2$ in 25 ml of $H_2O$. The mixture was stirred at 90°C for 20 hours, whereafter it was filtered, and evaporated to dryness *in vacuo*. The crystalline residue was suspended in $Et_2O$ and filtered off.

Yield: 26.5 g

NMR: ($CDCl_3$) $\delta$ (ppm) 5.20 (2H,s) 7.10 (2H,m) 7.40 (5H,s) 8.20 (2H,m) 9.65 (1H,s)

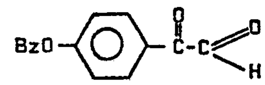

d) Preparation of 4-Pivaloyloxyphenylglyoxal monoethylacetal utilized in Method C.

*Step 1:* 4-Pivaloyloxyacetophenone

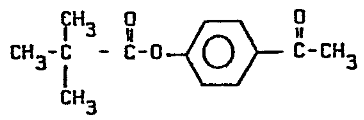

To a solution of 20 g (0.15 mole) p-hydroxy acetophenone in 200 ml of pyridine, 30 g (0.25 mole) of pivalic acid chloride is added dropwise with chilling and stirring. The mixture is then stirred for 20 hours at room temperature. After evaporation to dryness, the residue is partitioned between $Et_2O$ and water. The $Et_2O$-phase is washed with 2 N HCl, and then with water.

After evaporation of the $Et_2O$-phase, the residue is recrystallized from petroleum ether (bp 40—60°C).

Yield: 28 g

NMR: ($CDCl_3$) $\delta$ (ppm) 1.40 (9H,s) 2.65 (3H,s) 7.25 (2H,d) 8.10 (2H,d)

*Step 2:* 4-Pivaloyloxyphenylglyoxal monoethylacetal

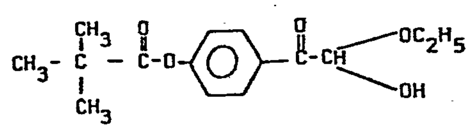

To a solution of 28 g (0.13 mole) 4-pivaloyloxyacetophenone in 200 ml of dioxane was added a solution of 16.7 g (0.15 mole) $SeO_2$ in 25 ml of $H_2O$. The mixture was stirred at 90°C for 20 hours, whereafter the solution was filtered and evaporated. The residue was refluxed with EtOH which was then evaporated off. A reddish oily residue was obtained.

Yield: 10 g

NMR: ($CDCl_3$) $\delta$ (ppm) 1.25 (3H,t) 1.40 (9H,s) 4.00 (3H,m) 5.70 (1H,s) 7.15 (1H,d) 8.18 (1H,d)

e) Preparation of 4-[4-methylbenzoyloxy]phenylglyoxal monoethylacetal

*Step 1:* 4-[4-methylbenzoyloxyl-acetophenone

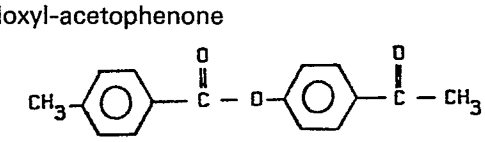

was prepared in the same manner as described for 4-pivaloyloxyacetophenone.

Yield: 71%

NMR: ($CDCl_3$) $\delta$ (ppm) 2.45 (6H,s) 7.35 (4H,m) 8.10 (4H,m)

*Step 2:* 4-[4-methylbenzoyloxy]phenylglyoxal monoethylacetal was prepared in the same manner as 4-pivaloyloxyphenyl glyoxalmonoethyl acetal.

Yield: 30%

NMR: ($CDCl_3$) $\delta$ (ppm) 1.25 (3H,t) 2.45 (3H,s) 3.80 (2H+1H,m) 7.80 (8H,m)

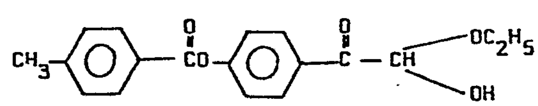

f) Preparation of 1-(4-benzyloxyphenyl)-2-[1,1-dimethyl-3-(2-ethoxyphenyl)propylamino]-ethanol utilized in Example 6

*Step 1:* 2-Ethoxybenzalacetone

The title compound was prepared from 2-ethoxybenzaldehyde and acetone in the same manner as described above for 2-methoxybenzalacetone.

Yield: 76%

NMR: ($CDCl_3$) $\delta$ (ppm) 1.4 (3H,t) 2.2 (3H,s) 4.0 (2H,q) 6.7 (1H,d) 7.2 (4H,m) 7.9 (1H,d)

*Step 2:* 4-[2-ethoxyphenyl]-buten-2-on

The title compound was prepared from 2-ethoxybenzalacetone in the same manner as described above for 4-[2-methoxyphenyl]-butan-2-on.

Yield: 68% B.p. 83—86°C/9.3 Pa (0.07 mm Hg)

NMR: ($CDCl_3$) $\delta$ (ppm) 1.15 (3H,t) 2.0 (3H,s) 2.7 (4H,m) 3.9 (2H,q) 6.9 (4H,m)

*Step 3:* 1,1-Dimethyl-3-[2-ethoxyphenyl]propanol

The title compound was prepared from 4-[2-ethoxyphenyl]-butan-2-on in the same manner as described above for 1,1-dimethyl-3-[2-methoxyphenyl]propanol.

Yield: 85%

NMR: ($CDCl_3$) $\delta$ (ppm) 1.2 (6H,s) 1.3 (3H,t) 1.7 (2H,m) 2.7 (2H,m) 4.0 (2H,q) 7.0 (4H,m)

*Step 4:* 1,1-Dimethyl-3-[2-ethoxyphenyl]-propylamine

The title compound was prepared from 1,1-dimethyl-3-[2-ethoxyphenyl]propanol in the same manner as described above for 1,1-dimethyl-3-[2-methoxyphenyl]-propylamine.

Yield: 15%

NMR: ($CDCl_3$) $\delta$ (ppm) 1.0 (6H,s) 1.2 (3H,t) 1.5 (2H,m) 2.6 (2H,m) 3.9 (2H,q) 6.9 (4H,m)

*Step 5:* 1-(4-Benzyloxyphenyl)-2-[1,1-dimethyl-3-(2-ethoxyphenyl)propylamino]-ethanol

The title compound was prepared from 4-benzyloxyphenylglyoxal and 1,1-dimethyl-3-[2-ethoxyphenyl]-propylamine as described in Example 3a.

Yield: 15%

NMR: ($CDCl_3$) $\delta$ (ppm) 1.2 (6H,s) 1.5 (3H,t) 1.7 (2H,m) 2.8 (2H,m) 3.8 (2H,m) 4.1 (2H,q) 4.8 (1H,m) 5.1 (2H,s) 7.2 (13h,m)

g) Preparation of 1-(4-benzyloxyphenyl)-2-[1,1-dimethyl-4-(2-methoxyphenyl)butylamino]ethanol utilized in Example 7

*Step 1:* *o*-methoxyphenylpropionic acid

A mixture of 68 g of *o*-methoxybenzaldehyde, 75 g acetic anhydride, and 30 g sodium acetate was heated with stirring at 150°C for 1 hour and thereafter at 170—180°C for 15 hours. The mixture was then cooled to 90°C and poured into 400 ml $H_2O$. The resulting mixture was neutralized with $Na_2CO_3$, and then steam-distilled until a clear solution resulted, which was then filtered hot. The filtrate was acidified with conc. HCl, and the precipitate (51.2 g) was collected and washed with water. The precipitate was dissolved in 300 ml $CH_3OH$ and hydrogenated at room temperature, and atmospheric pressure in the presence of 0.3 g Pd/C. When the calculated amount hydrogen had been taken up the mixture was filtered, and evaporated to give 50.2 g of the title compound.

NMR: ($CDCl_3$) $\delta$ (ppm) 2.8 (4H,m) 3.8 (3H,s) 7.1 (4H,m) 11.4 (1H,s)

*Step 2:* *o*-methoxyphenylpropanol

A solution of 50 g *o*-methoxyphenyl propionic acid in 500 ml of dry ethyl ether was added dropwise to a chilled, and stirred slurry of 10 g $LiAlH_4$ in dry ether. The reaction mixture was then slowly allowed to reach room temperature whereafter it was neutralized with a 20% K-Na-tartrate solution at 0°C. The etherphase was decanted, and the residue washed with ether. The combined ether phases were washed with $H_2O$, dried over $MgSO_4$, and evaporated to yield the title compound.

Yield: 38.2 g

NMR: ($CDCl_3$) $\delta$ (ppm) 1.8 (2H,t) 2.1 (1H,s) 2.7 (2H,t) 3.6 (2H,t) 3.8 (3H,s) 7.0 (4H,m)

*Step 3:* *o*-methoxyphenylpropylchloride

To a solution of 38 g *o*-methoxyphenylpropanol in 100 ml of trichloroethylene was added dropwise 60 ml thionylchloride. The solution was boiled under reflux for 3 hours whereafter it was evaporated. The residue was distilled, and the fraction boiling 122—126°C/17 mm Hg was collected.

Yield: 21.6 g

NMR: ($CDCl_3$) $\delta$ (ppm) 2.2 (2H,m) 2.8 (2H,m) 3.5 (2H,m) 3.8 (3H,s) 7.1 (4H,m)

*Step 4:* 1,1-Dimethyl-4-(2-methoxyphenyl)butanol

A solution of 21.6 g of *o*-methoxyphenylpropyl chloride in 10 ml of dry ethyl ether was added dropwise to a mixture of 3.0 g Mg in 10 ml of dry ether. When formation of the Grignard reagent was completed, 7 g of acetone was added. The reaction was allowed to proceed for 2 hours at room temperature, whereafter the mixture was chilled, and water added.

The ether phase was separated, washed with $H_2O$, dried over $MgSO_4$, and evaporated to yield 20.6 g of the title compound.
NMR: ($CDCl_3$) 1.1 (6H,s) 1.4 (4H,m) 2.5 (2H,m) 3.8 (3H,s) 7.0 (4H,m)

*Step 5:* 1,1-Dimethyl-4-(2-methoxyphenyl)butylamine

The title compound was prepared from 20.5 g 1,1-dimethyl 4-(2-methoxyphenyl)butanol in the same way as described above for 1,1-dimethyl-3-[2-methoxyphenyl]-propylamine.
Yield: 14.1 g base
Yield: 2.8 g sulfate of title compound
NMR: ($CDCl_3$) 1.1 (6H,s) 1.5 (4H,m) 2.7 (2H,m) 3.8 (3H,s) 7.0 (4H,s)

*Step 6:* 1-(4-Benzyloxyphenyl)-2-[1,1-dimethyl-4-(2-methoxyphenyl)butylamino]-ethanol sulfate

The title compound was prepared from 2.4 g 1,1-dimethyl-4-(2-methoxyphenyl)butylamine, and 3.0 g 4-benzyloxyphenyl glyoxal in the same way as described in Example 3a.
Yield: 2.6 g
NMR: ($CDCl_3$ + $CF_3COOH$) $\delta$ (ppm) 1.1 (6H,s) 1.5 (4H,m) 2.5 (2H,m) 2.9 (2H,m) 3.7 (3H,s) 5.0 (2H,s) 5.1 (1H,m) 7.0 (13H,m)

h) Preparation of 1-(4-benzyloxyphenyl-2-[1,1-dimethyl-3-(2-hydroxyphenyl)propylamino]-ethanol utilized in Example 8

*Step 1:* 1,1-Dimethyl-3-[2-hydroxyphenyl]-propylamine

1,1-Dimethyl-3-[2-methoxyphenyl]-propylamine, 3.0 g was dissolved in 100 ml 48% HBr, and boiled under reflux for 1 hour, whereafter 50 ml of HBr was added. The mixture was boiled for 15 hours whereafter the HBr was evaporated *in vacuo*. The residue was dissolved in $H_2O$, and treated with activated carbon. The water phase was then made alkaline, and the amine extracted into ether. Evaporation of the ether phase gave an oily residue.
Yield: 2.1 g
NMR: ($D_2O$) $\delta$ (ppm) 1.15 (6H,s) 1.8 (2H,m) 2.6 (2H,m) 4.8 (DOH) 7.1 (4H,m)

*Step 2:* 1-(4-Benzyloxyphenyl)-2-[1,1-dimethyl-3-(2-hydroxyphenyl)propylamino]-ethanol sulfate

The title compound was prepared from 1.35 g 1,1-dimethyl-3-[2-hydroxyphenyl]-propylamine, and 4-benzyloxyphenyl glyoxal, 2.2 g in the same way as described in Example 3a.
Yield: 1.9 g
NMR: ($CD_3OD$) $\delta$ (ppm) 1.2 (6H,s) 1.6 (2H,m) 2.4 (2H,m) 2.9 (2H,m) 3.1 ($CD_3OD$) 4.7 (1H,m) 4.8 (2H,s) 6.8 (13H,m)

Starting materials in method B may be prepared according to the following reaction schemes:
a) reacting a compound of the formula

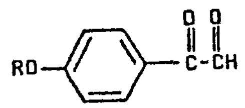

wherein R has the meaning indicated in method A above, with a compound of the formula

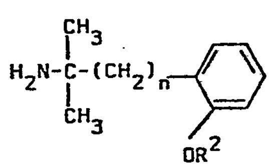

wherein n, $R^2$ and $R^5$ have the meaning indicated under A above, to the formation of a compound of the formula

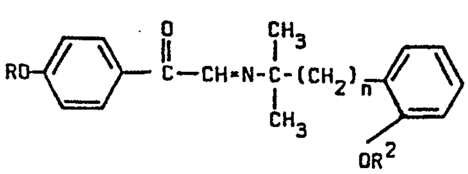

b) reacting a compound of the formula

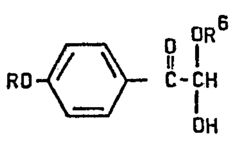

wherein R has the meaning indicated under A above, and wherein $R^6$ is hydrogen or an alkyl group containing 1—5 carbon atoms, with a compound of the formula

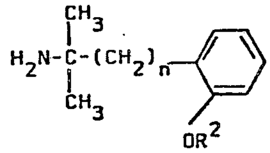

wherein n and $R^2$ have the meaning indicated in Method A above, to the formation of a compound of the formula

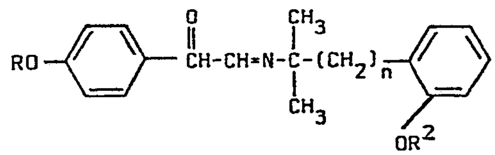

The following examples illustrate how the compounds of the invention can be incorporated in pharmaceutical compositions:

Example 9

*Aerosol for inhalation*

| | | |
|---|---|---|
| 1-(4-hydroxyphenyl)-2-[1,1-dimethyl-3-(2-methoxyphenyl) propylamino]ethanol sulfate | | 0.25 g |
| Miglyol® | | 0.20 g |
| Frigen® 11/12/113/114 | ad | 100.0 g |

Example 10

*Tablets*

Each tablet contains:

| | |
|---|---|
| 1-(4-hydroxyphenyl)-2-[1,1-dimethyl-3-(2-methoxyphenyl) propylamino]-ethanol sulfate | 2.0 mg |
| Maize starch | 25.0 mg |
| Lactose | 210.0 mg |
| Gelatin | 1.5 mg |
| Talc | 10.0 mg |
| Magnesium stearate | 1.5 mg |
| | 250.0 mg |

Example 11

*Suppositories*

Each suppository contains:

| | |
|---|---|
| 1-(4-hydroxyphenyl)-2-[1,1-dimethyl-3-(2-methoxyphenyl) propylamino]-ethanol sulfate | 2.0 mg |
| Ascorbyl palmitate | 1.0 mg |
| Suppository base (Imhausen H) | ad 2.000.0 mg |

Example 12

*Syrup*

| | |
|---|---|
| 1-(4-hydroxyphenyl)-2-[1,1-dimethyl-2-(2-methoxyphenyl) ethylamino]ethanol sulfate | 0.020 g |
| Liquid glucose | 30.0 g |
| Sucrose | 50.0 g |
| Ascorbic acid | 0.1 g |
| Sodium pyrosulfite | 0.01 g |
| Disodium edetate | 0.01 g |
| Orange essence | 0.025 g |
| Certified colour | 0.015 g |
| Purified water | ad 100.0 ml |

Example 13

*Injection solution*

| | |
|---|---|
| 1-(4-hydroxyphenyl)-2-[1,1-dimethyl-3-(2-methoxyphenyl) propylamino]-ethanol hydrochloride | 0.200 mg |
| Sodium pyrosulfite | 0.500 mg |
| Disodium edetate | 0.100 mg |
| Sodium chloride | 8.500 mg |
| Sterile water for injection | ad 1.00 ml |

Example 14

*Inhalation solution*

| | |
|---|---|
| 1-(4-hydroxyphenyl)-2-[1,1-dimethyl-3-(2-methoxyphenyl) propylamino]-ethanol sulfate | 0.25 g |
| Sodium pyrosulfite | 0.10 g |
| Disodium edetate | 0.10 g |
| Sodium chloride | 0.85 g |
| Purified water | ad 100.0 ml |

Example 15

*Solution for rectal administration (Rectal vials)*

| | |
|---|---|
| 1-(4-hydroxyphenyl)-2-[1,1-dimethyl-3-(2-methoxyphenyl) propylamino]-ethanol sulfate | 2.0 mg |
| Sodium pyrosulfite | 1.5 mg |
| Disodium edetate | 0.3 mg |
| Sterile water | ad 3.0 ml |

Example 16

*Sublingual tablets*

| | | |
|---|---|---|
| 1-(4-hydroxyphenyl)-2-[1,1-dimethyl-3-(2-methoxyphenyl) propylamino]-ethanol hydrochloride | | 1.0 mg |
| Lactose | | 85.0 mg |
| Agar | | 5.0 mg |
| Talc | | 5.0 mg |
| | | 100.0 mg |

Example 17

*Drops*

| | | |
|---|---|---|
| 1-(4-hydroxyphenyl)-2-[1,1-dimethyl-3-(2-methoxyphenyl) propylamino]-ethanol sulfate | | 0.20 g |
| Ascorbic acid | | 1.00 g |
| Sodium pyrosulfite | | 0.10 g |
| Disodium edetate | | 0.10 g |
| Liquid glucose | | 50.00 g |
| Absolute alcohol | | 10.00 g |
| Purified water | ad | 100.0 ml |

*Pharmacological tests*

A. Simultaneous recording of bronchospasmolytic effect, tremorogenic effect and heart stimulating effect

The bronchospasmolytic effect and the tremorogenic effect were measured simultaneously in anaesthetized cat.

*Method:*

The method used is described in detail by O A T Olsson, Acta pharmacol et toxicol *34,* 106—114 (1974).

Cats weighing 2.5—3.5 kg are anaesthetized with pentobarbital (mebumalum NFN) at first by an intraperitoneal injection (30 mg/kg) and afterwards by continuous intravenous infusion to keep the level of anaesthesia constant during the experiment.

Arterial blood pressure is measured with a Statham transducer from a cannulated carotid artery. Heart frequency is monitored by means of a tachometer set to trigger off the R-wave of the electrocardiogram.

The soleus muscle in the hind limb, consisting mainly a slow contracting fibres, is prepared essentially according to Bowman & Zaimis, J Physiol *144,* 92—107 (1958). The muscle is enclosed in a chamber made of a rubber covered plastic "hair-curler". The entire leg is wrapped in metal foil covered with plastic to keep the temperature constant at 35—36°C (controlled by thermo couples in the leg). The cat is placed on its back on a thermostatically controlled operation table and the right hind limb fixed, special care being taken not to impede the blood flow. The tendon is cut and attached to a Grass strain gauge (FT 10C). The isometric tension of the muscle is calibrated to 50 g (resting tension) and 500 g (maximal tension). A plexiglas shielded bipolar Pt-electrode is placed on the tibialis nerve, near the soleus. The incomplete tetanic contractions of the muscle are elicited by a square wave pulse stimulation of the nerve by the use of a Grass S48 stimulator. The pulse duration is 0.05 msec, with frequencies selected in the range 8—12 Hz and the strength between 5—8 volts. The stimulation occurs for 1.8 sec every 20 sec.

The trachea is cannulated and the lungs inflated with room air by means of a constant volume Braun-respirator. The frequency is 15—20 per min and the tidal volume 8—10 ml per kg, or just sufficient to suppress spontaneous breathing movements and to give about 150 ml/kg/min. The change in tidal volume from administered constricting agents is measured as the amount of air overflow from

the delivery of a constant volume to a fixed respiration. The pressure is determined by a water (60—70 mm) pressure threshold and respiratory overflow determined by means of a Grass-volumetric pressure transducer PT 5A. Bronchial tone is increased by inhalation of a histamine aerosol generated in a Roth-Petersen nebulizer from a solution containing 0.1—0.3 mg/ml of histamine and made up to 5% by glycerol. The nebulizer is connected to the inlet of the respirator when bronchospasm is to be produced. When the respiratory overflow has reach an even level, the compound to be tested for bronchorelaxing activity is injected into the brachial vein.

*Test results*

The test results are given in Table 1. The bronchospasmolytic effect is measured as the dose, calculated in moles per kg bodyweight of the test animal, which gives 30% decrease of the histamine induced bronchospasm. The tremorogenic effect is measured as the dose, also calculated in moles per kg bodyweight of the test animal, which gives 30% decrease of the electrically induced contractions of the soleus muscle. The heart stimulating effect is measured as the dose, calculated in moles per kg bodyweight of the test animal, which gives 25% increase in the heart frequency.

The effect of the compounds are given in relation to the effects of terbutaline, a clinically used bronchospasmolytic agent which has a selective effect on the bronchi and exhibits a very small heart-stimulating effect.

TABLE 1

Simultaneous recording of bronchospasmolytic effect, tremorogenic effect and heart stimulating effect in narcotized cat

| Test Compound 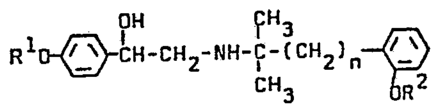 | | | | Bronchospasmolytic effect in relation to the bronchospasmolytic effect of terbutaline | Tremorogenic effect in relation to the tremorogenic effect of terbutaline | Heart stimulating effect in relation to the heart stimulating effect of terbutaline |
|---|---|---|---|---|---|---|
| $R^1$ | $R^2$ | n | Code | | | |
| H | $CH_3$ | 2 | D 2343 | 2.9 ± 0.6 | 0.5 ± 0.1 | 0.9 ± 0.1 |
| H | $CH_3$ | 1 | D 2331 | 0.12 ± 0.03 | 0.011 ± 0.004 | 0.02 |
| H | $C_2H_5$ | 2 | D 2388 | 1.4 ± 0.2 | 0.8 ± 0.2 | 1 |
| 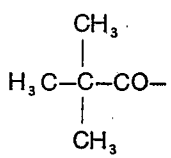 | $CH_3$ | 2 | D 2367 | 0.28 ± 0.07 | 0.23 ± 0.07 | 0.16 ± 0.05 |
| 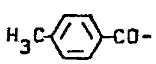 | $CH_3$ | 2 | D 2375 | 0.25 ± 0.05 | 0.18 ± 0.03 | 0.26 ± 0.04 |
| Terbutaline | | | | 1 | 1 | 1 |

*Comments to the test results*

It is seen in Table 1 that the tested compounds in relation to the terbutaline exhibit a relatively higher bronchospasmolytic effect than tremorogenic effect. This is particularly pronounced for the compounds D 2343 and D 2331. The compound D 2343, which exhibits a bronchospasmolytic effect which is almost 3 times the bronchospasmolytic effect of terbutaline while having a tremorogenic effect which is only 0.5 times the tremorogenic effect of terbutaline, and a heart stimulating effect which does not exceed the heart stimulating effect of terbutaline, is the preferred compound of the invention.

B. Bronchospasmolytic activity at oral administration and at subcutaneous administration

The bronchospasmolytic activity of a compound of the invention at oral administration was tested on guinea-pigs. The compound according to Example 1 having the code designation D 2343, was tested. Terbutaline was used as reference substance.

*Method:*

The bronchospasmolytic effect was tested as the inhibitory effect on histamine induced bronchoconstriction in unanaesthetized guinea-pigs.

The test animals were exposed to a histamine mist generated by a Roth-Petersen nebulizer from a solution of 0.3 mg/ml of histamine-chloride.

Guinea-pigs, strain Dunkin-Hartley, both sexes, 190—250 g were used. Saline treated control animals start a forced and irregular breathing after less than 4 minutes exposure to the aerosol. Drug-treated animals sustaining the histamine aerosol 4 minutes without a breathing influence are regarded as protected. In the study with oral administration, the animals were starved for about 15 hours with water ad libitum before D 2343 or terbutaline was administered by a stomach tube.

In the study with subcutaneous administration, 3—4 different dose levels of D 2343 and terbutaline were administered 15 minutes before the histamine challenge to determine $ED_{50}$, which is the dose protecting 50% of the animals for more than 4 minutes in the histamine aerosol. Each compound was administered to 30—40 animals.

In the investigation with oral administration the time from drug administration to exposure of the animal to histamine was 30 minutes.

*Results*

The test results are given in Table 2. The $ED_{50}$ doses are calculated on molar basis in moles per kg bodyweight of the test animal.

TABLE 2

Bronchospasmolytic effect of 1-(4-hydroxyphenyl)-2-[1,1-dimethyl-3-(2-methoxyphenyl)-propylamine]-ethanol at oral and subcutaneous administration

| | Dose protecting 50 % of the test animals against the histamine aerosol during more than 4 minutes ($ED_{50}$) μmoles /kg | |
|---|---|---|
| Test Compound | Subcutaneous administration | Oral administration |
| 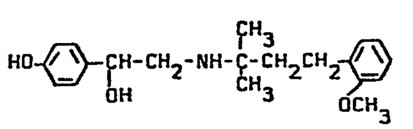 | 0.34 ± 0.06 | 2.51 ± 0.39 |
| Terbutaline | 0.93 ± 0.14 | 5.28 ± 0.86 |

*Comments to the test results*

It is seen in Table 2 that the tested compound of the invention (D 2343) was 2.7 times more active than terbutaline at subcutaneous administration and 2.1 times more active than terbutaline at oral administration.

C. Onset and duration of bronchospasmolytic activity after oral administration

The onset and the duration of bronchospasmolytic activity of the compounds according to Example 1 and Example 4 was tested. These compounds have the structural formulas

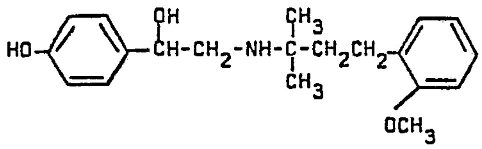 (Compound of Example 1)

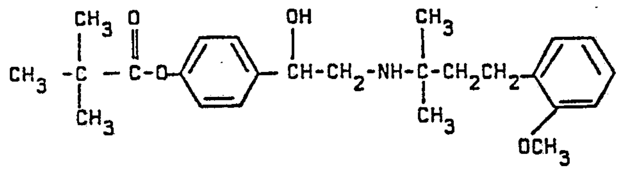 (Compound of Example 4)

*Method*

The bronchospasmolytic effect was tested as the inhibitory effect on histamine induced bronchoconstriction in unanaesthetized guinea-pigs.

The test animals were exposed to a histamine mist generated by a Roth-Petersen nebulizer from a solution of 0.3 mg/ml of histamine-chloride.

Guinea-pigs, strain Dunkin-Hartley, both sexes, 160—230 g were used. Saline treated control animals start a forced and irregular breathing after less than 4 minutes exposure to the aerosol. Drug-treated animals sustaining the histamine aerosol 4 minutes without a breathing influence are regarded as protected. The animals were starved for about 15 hours with water ad libitum before the test compound was administered by a stomach tube. Each of the test substances was given in an amount of $4.10^{-6}$ mole/kg bodyweight. Groups of 8—10 animals were tested in the aerosol at 7, 15, 30, 60 and 120 minutes after administration of the test substance. The percentage of the animals which could withstand the aerosol for 4 minutes and for 10 minutes was determined. The results are given in Table 3.

TABLE 3

Onset and duration of bronchospasmolytic activity at oral administration

| Test Compound | Percentage animals protected against the histamine aerosol during ≥ 4 min and ≥ 10 min at (min) | | | | | | | | | |
|---|---|---|---|---|---|---|---|---|---|---|
| | 7 | | 15 | | 30 | | 60 | | 120 | |
| | ≥4 min | ≥10 min | ≥4 min | ≥10 min | ≥4 min | ≥10 min | ≥4 min | ≥10 min | ≥4 min | ≥10 min |
| 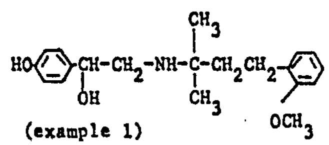 (example 1) | 50 | 17 | 75 | 63 | 50 | 13 | 50 | 0 | 1 | 0 |
| 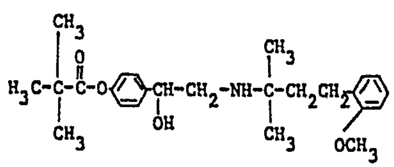 (example 4) | 33 | | 60 | 20 | 50 | 10 | 20 | 10 | 29 | 15 |

It is seen in Table 3 that both of the tested compounds give a rapid onset.

The duration of the compound of example 4 was considerably prolonged compared to the duration of the compound of example 1.

D. Test on α-receptor blocking activity

The α-receptor blocking activity of two compounds of the invention was investigated. This effect is relevant in the treatment of asthma. It has been shown, e.g. by Nousiainen et al, Pharmacology *15*, 469—477 (1977), that certain compounds having α-blocking effect may potentiate the effects of β-receptor-stimulating agents such as terbutaline.

In the test, the α-receptor blocking substance thymoxamine was used as reference.

*Method:*

Albino rabbits of both sexes (2—3 kg) were used. About 5 cm of the aorta was dissected out as near the arch as possible, transferred to a dish with Krebs solution and cut spirally. Usually 3 preparations were received from the dissected piece of vessel. Each strip was mounted in an organ bath in Krebs solution of 37°C and aerated with carbogen. The load on the preparation was 2 g and the tension changes recorded isometrically on Grass transducer FRO3 and polygraph P5.

Noradrenaline, which is a strong α-receptor stimulating agent, exerts a contracting effect on the aortic strips when added to the bath. The α-receptor blocking effect of the test compound is measured by adding the test compound to the organ bath before adding noradrenaline.

Noradrenaline solution with a concentration producing 70—80% of maximal contraction (0.03—0.06 μg/ml) was used. The test compound was added to the organ bath 10 minutes before the addition of noradrenaline.

The results are measured as the concentration of the test compound which inhibits the noradrenaline-induced contraction by 50% (EC 50).

*Results*

The test results are given in Table 4 below.

TABLE 4

α-receptor blocking activity of the compounds of the invention

| Test Compound | Concentration inhibiting 50 % noradrenaline-induced contraction μM |
|---|---|
| 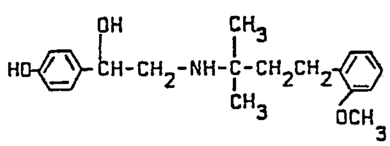 | 0.6 ± 0.2 |
| 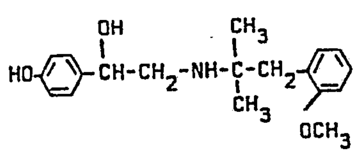 | 0.5 ± 0.2 |
| Thymoxamine | 1.5 ± 0.4 |

The EC 50 values are based on tests on 6 preparations obtained from 3 animals.

*Comments to the test results*

It is seen in Table 4 that the compound D 2343 of the invention has an α-receptor blocking activity which is higer than the α-blocking effect of thyoxamine.

It has also been shown that the α-blocking effect of D 2343 is obtained in the same dose range as its β-stimulating effect. As pointed out previously, the combination of α-adrenoceptor blocking properties and β-adrenoceptor stimulating properties may be beneficial in the treatment of asthma.

E. Inhibitory activity on oxytocin and carbachol induced contractions of rat uterus in vitro

The uterus relaxing effect of the compounds of the invention was tested. Terbutaline was used as reference substance.

*Method:*

Female Sprague-Dawley rats, 150—200 g, were used. An oestrogen steroid (Estradurin, Leo), 0.25 mg/100 g, was administered s.c. 4—6 days before the experiments to bring the animals in oesterus.

The uterus horn were cut open longitudinally, mounted in organ baths in Locke solution at 33°C and gassed with carbogen. The contraction of the preparation was produced by oxytocin (Partocon, Ferring) or carbachol and given to the bath 3 min after administration of the test substance in a dose producing 70—80% of the maximum contraction or 0.005—0.02 IE/ml of oxytocin and 0.2—0.6 μg/ml of carbacholine chloride. The inhibitory effect produced by the test substance was eliminated by propranolol (1 μg/ml or $0.4 \times 10^{-5}$ M given to the bath 15 minutes before the drugs).

The tension changes were recorded isometrically (transducer FPO3 and Grass polygraph P5).

*Results*

The results are given in Table 5. The results are given as the concentration of the test compound which reduces the contraction induced by oxytocin and carbachol, respectively, by 50% (EC 50).

TABLE 5

Inhibiting effect on induced contractions of rat uterus

| Test Compound | Inhibition of contractions induced by oxytocin EC 50 (M) | Inhibition of contractions induced by carbachol EC 50 (M) |
|---|---|---|
| 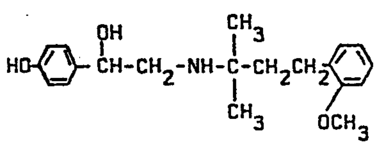 | $0.18 \pm 0.06 \times 10^{-8}$ 1) | $0.32 \pm 0.08 \times 10^{-8}$ 2) |
| Terbutaline | $2.10 \pm 0.70 \times 10^{-8}$ 1) | $2.61 \pm 0.51 \times 10^{-8}$ 2) |

1) p = 0.01

2) p = 0.001

Oxytocinseries: 10 preparations from 6 animals

Carbacholseries: 9 preparations from 5 animals

*Comments to the test results*

It is seen in Table 5 that the compound D 2343 of the invention is more potent as uterus relaxing agent than terbutaline. Terbutaline has been tested as uterus relaxing agent in order to inhibit premature labor, e.g. by Andersson et al., Acta Obstet. Gynec. Scand. 1974. The test results of Table 4 indicate that the compound D 2343 of the invention is at least as effective as terbutaline in the use as

uterus relaxing agent. It is of particular importance for the use as uterus relaxing agent to have access to a compound which has reduced tremorogenic activity, because the medication in such cases is given during a limited period of time only. It has been found at treatment with terbutaline that the tremor occurring at the start of the treatment subsequently is reduced when the treatment has continued for some time. Such a regression of the tremorogenic effect is not obtained at treatment for relaxing the uterus, because of the short duration of the medication in such cases. The compounds of the present invention represent therefore in that respect an important improvement at treatment aiming to relax the human uterus.

E. Acute toxicity in mice

The acute toxicity of the compound D 2343 of the invention was investigated.

Male mice (NMRI) 20—25 g were used. The test compound was administered intravenously (by tail vein), subcutaneously, intraperitoneally and orally (by stomach tube to starved animals) in 6 dose levels in log-sequence with 10 or more animals tested at each dose level to determine the $LD_{50}$.

After toxic doses the death followed within the first hour after i.v., s.c. and i.p. and during the first three hours after p.o. administration. No further mortality was noted during the following 5 days the animals were observed. The results are given in Table 6.

TABLE 6

Acute toxicity of the compound D 2343 of the invention

| | $LD_{50}$ | |
|---|---|---|
| | D 2343 (M = 378.5) | |
| Route of administration | mg/kg | M/kg ($\times 10^{-5}$) |
| i.v. | 13 (±1) | 3.5 |
| s.c. | 50 (±2) | 13 |
| i.p. | 45 (±3) | 12 |
| p.o. | 170 (±8) | 45 |

F. Use as $\alpha$-receptor blocking agent

The $\alpha$-receptor blocking properties of the compounds of the inventions mean that the compounds may be used for the treatment of Raynaud's disease. Raynaud's disease is a paroxysmal disorder of the arteries of the fingers and toes characterized by attacks of pain in them, the fingers or toes going white and then blue.

**Claims**

1. Ethanolamine compounds of the formula

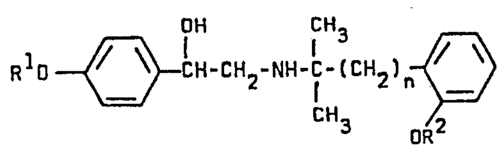

I

and therapeutically acceptable salts thereof, in which formula n is 1, 2 or 3; $R^1$ is hydrogen, an aliphatic acyl group containing 2 to 5 carbon atoms, or benzoyl of the formula

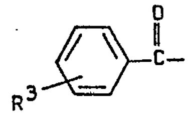

wherein $R^3$ is hydrogen or methyl; and wherein $R^2$ is hydrogen, an aliphatic alkyl group containing 1—4 carbon atoms, benzyl, an aliphatic acyl group containing 2—5 carbon atoms, or benzoyl of the formula

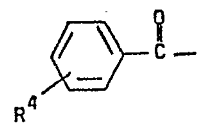

wherein $R^4$ is hydrogen or methyl.

2. Compounds according to claim 1 and therapeutically acceptable salts thereof, wherein $R^1$ is hydrogen.

3. Compounds according to claim 1 and therapeutically acceptable salts thereof, wherein $R^1$ is an aliphatic acyl group containing 2—5 carbon atoms or benzoyl of the formula

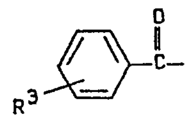

wherein $R^3$ is hydrogen or methyl.

4. Compounds according to claim 1 and therapeutically acceptable salts thereof, wherein n is 1.

5. Compounds according to claim 1 and therapeutically acceptable salts thereof, wherein n is 2.

6. Compounds according to claim 1 and therapeutically acceptable salts thereof, wherein $R^2$ is an alkyl group containing 1—4 carbon atoms.

7. Compounds according to claim 1 and therapeutically acceptable salts thereof, wherein n is 2, $R^1$ is hydrogen, and $R^2$ is an alkyl group containing 1—4 carbon atoms.

8. Compounds according to claim 1 and therapeutically acceptable salts thereof, wherein n is 2, $R^1$ is an aliphatic acyl group containing 2—5 carbon atoms, and $R^2$ is an alkyl group containing 1—4 carbon atoms.

9. A compound of the formula

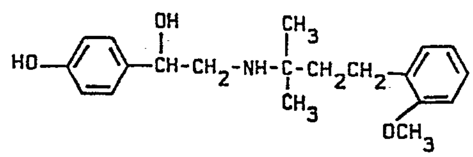

and therapeutically acceptable salts thereof;

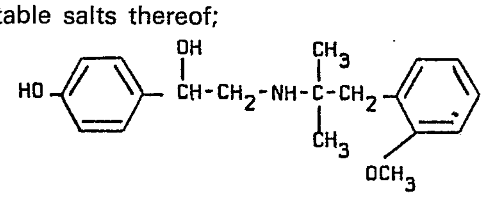

and therapeutically acceptable salts thereof;

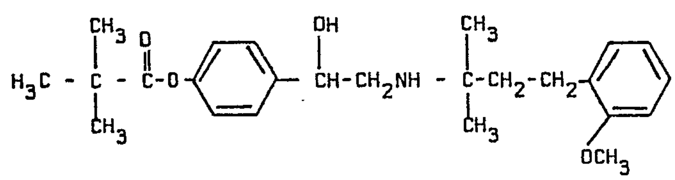

and therapeutically acceptable salts thereof; or

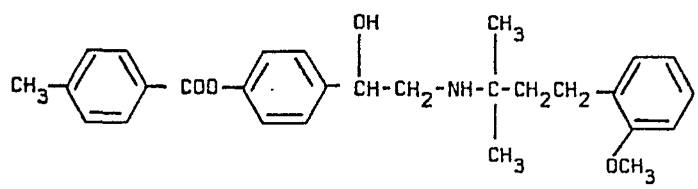

and therapeutically acceptable salts thereof.

10. A compound of the formula

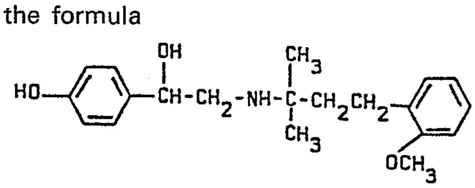

and therapeutically acceptable salts thereof.

11. A compound according to any of claims 1—10 in the form of
a) a racemic mixture or
b) a substantially pure stereoisomer.

12. A process for the preparation of the compounds of the formula

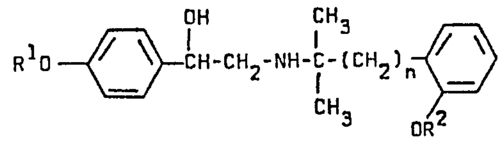

I

and therapeutically acceptable salts thereof, in which formula n is 1, 2 or 3; $R^1$ is hydrogen, an aliphatic acyl group containing 2 to 5 carbon atoms, or benzoyl of the formula

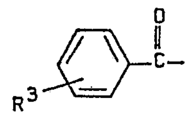

wherein $R^3$ is hydrogen or methyl; and wherein $R^2$ is hydrogen, an aliphatic alkyl group containing 1—4 carbon atoms, benzyl, an aliphatic acyl group containing 2 to 5 carbon atoms, or benzoyl of the formula

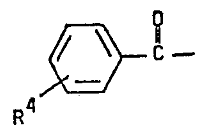

wherein $R^4$ is hydrogen or methyl, by

A. reacting a compound of the formula

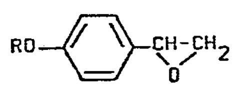

III

wherein R is hydrogen; an aliphatic acyl group containing 2 to 5 carbon atoms; benzoyl of the formula

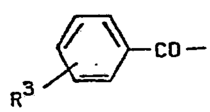

wherein $R^3$ is hydrogen or methyl; or a hydroxy-protecting group with a compound of the formula

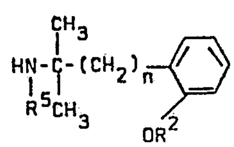

IV

wherein n is 1, 2 or 3; $R^2$ is hydrogen; an aliphatic alkyl group containing 1—4 carbon atoms; benzyl; an aliphatic acyl group containing 2 to 5 carbon atoms, or benzoyl of the formula

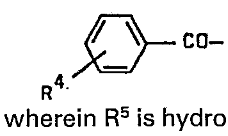

wherein $R^4$ is hydrogen or methyl; and wherein $R^5$ is hydrogen or a N-protecting group, to the formation of a compound of the formula

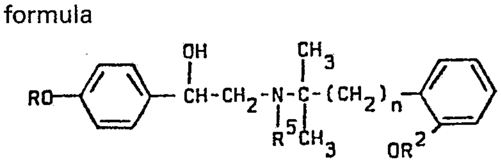

V

whereafter, if necessary, protecting groups R and $R^5$ are removed;

B. reducing a compound of the formula

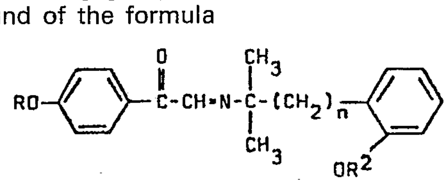

VI

wherein n, R and $R^2$ have the meanings indicated under A above, to the formation of a compound of the formula

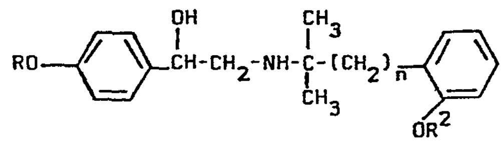

V

whereafter if necessary, the protecting group R is removed; whereafter, if desired, the compound of the formula I thus obtained is converted into a pharmaceutically acceptable salt and/or resolved into its optical isomers.

13. A pharmaceutical preparation comprising as active ingredient a compound of the formula

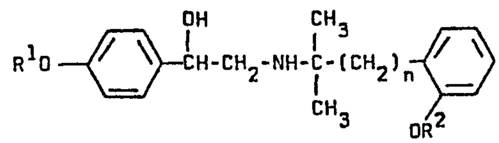

I

or a therapeutically acceptable salt thereof as defined in claim 1 in association with a pharmaceutically acceptable carrier.

14. A pharmaceutical preparation according to claim 13 in dosage unit form.

15. A compound according to any of claim 1—11, for use in producing bronchodilation in mammals including man.

16. A compound according to any of claims 1—11, for use in producing blockade of the $\alpha$-receptors in mammals including man.

17. A compound according to any of claims 1—11, for use in producing relaxation of the human uterus.

**Revendications**

1. Composés d'éthanolamine de formule:

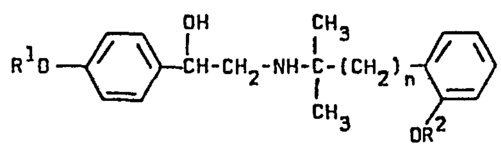

et leurs sels thérapeutiquement acceptables, formule dans laquelle *n* est 1, 2 ou 3; $R^1$ est l'hydrogène, un groupe acyle aliphatique contenant 2 à 5 atomes de carbone ou un groupe benzoyle de formule:

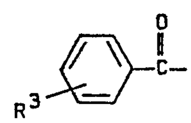

dans laquelle $R^3$ est l'hydrogène ou le groupe méthyle et $R_2$ est l'hydrogène ou un groupe alkyle aliphatique contenant 1 à 4 atomes de carbone, le groupe benzyle, un groupe acyle aliphatique contenant 2 à 5 atomes de carbone ou un groupe benzoyle de formule:

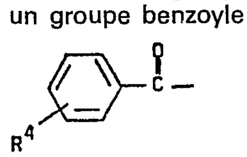

dans laquelle $R^4$ est l'hydrogène ou le méthyle.

2. Composés selon la revendication 1 et leurs sels thérapeutiquement acceptables, caractérisés en ce que $R^1$ est un atome d'hydrogène.

3. Composés selon la revendication 1 et leurs sels thérapeutiquement acceptables, caractérisés en ce que $R^1$ est un groupe acyle aliphatique contenant 2 à 5 atomes de carbone ou un groupe benzoyle répondant à la formule:

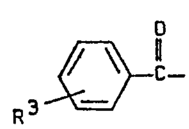

dans laquelle $R^3$ est un atome d'hydrogène ou le groupe méthyle.

4. Composés selon la revendication 1 et leurs sels thérapeutiquement acceptables, caractérisés en ce que n est 1.

5. Composés selon la revendication 1 et leurs sels thérapeutiquement acceptables, caractérisés en ce que n est 2.

6. Composés selon la revendication 1 et leurs sels thérapeutiquement acceptables, caractérisés en ce que $R^2$ est un groupe alkyl contenant 1 à 4 atomes de carbone.

7. Composés selon la revendication 1 et leurs sels thérapeutiquement acceptables, caractérisés en ce que n est 2, que $R^1$ est un atome d'hydrogène et que $R^2$ est un groupe alkyle contenant 1 à 4 atomes de carbone.

8. Composés selon la revendication 1 et leurs sels thérapeutiquement acceptables, caractérisés en ce que n est 2, que $R^1$ est un group acyle aliphatique contenant 2 à 5 atomes de carbone et que $R^2$ est un groupe alkyle contenant 1 à 4 atomes de carbone.

9. Composés répondant aux formules:

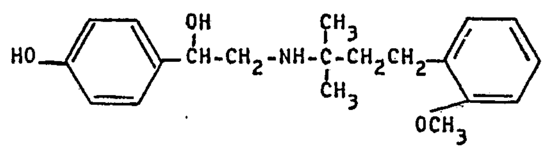

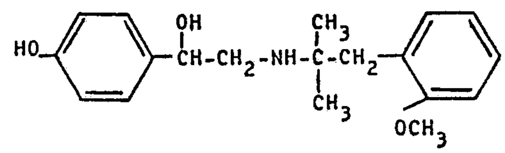

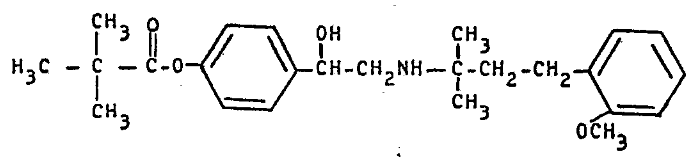

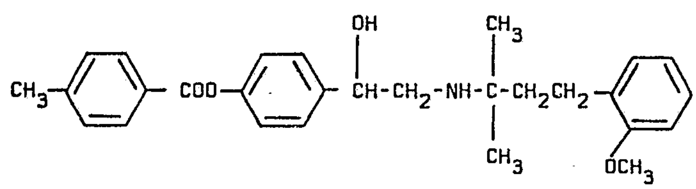

ainsi que leurs sels thérapeutiquement acceptables.

10. Composé de formule:

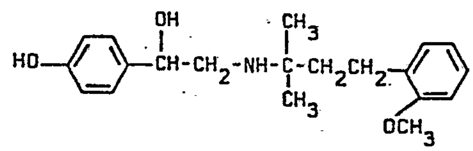

ainsi que ses sels thérapeutiquement acceptables.

11. Composés selon l'une quelconque des revendications 1 à 10, sous la forme:

a) d'un mélange racémique ou

b) d'un stéréoisomère pratiquement pur.

12. Procédé pour l'obtention de composés de formule:

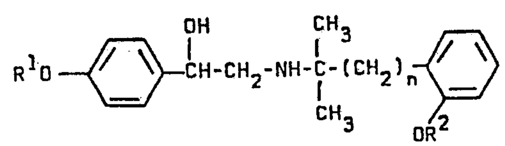

I

et leurs sels thérapeutiquement acceptables, formule dans laquelle n est 1, 2 ou 3; $R^1$ est l'hydrogène ou un groupe acyle aliphatique contenant 2 à 5 atomes de carbone ou un groupe benzoyle de formule:

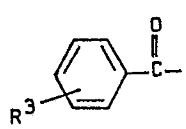

dans laquelle $R^3$ est l'hydrogène ou le groupe méthyle et $R^2$ est l'hydrogène ou un groupe alkyle aliphatique contenant 1 à 4 atomes de carbone, le groupe benzyle, un groupe acyle aliphatique contenant 2 à 5 atomes de carbone, ou un groupe benzoyle de formule:

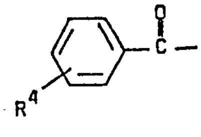

dans laquelle $R^4$ est l'hydrogène ou le groupe méthyle, caractérisé en ce qu'il consiste:

A) à faire réagir un composé de formule:

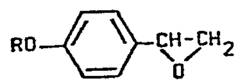

III

dans laquelle R est l'hydrogène, un groupe acyle aliphatique contenant 2 à 5 atomes de carbone, un groupe benzoyle de formule:

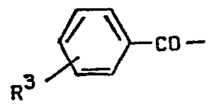

dans laquelle $R^3$ est l'hydrogène ou le méthyle ou un groupe protecteur d'hydroxy, avec un composé de formule:

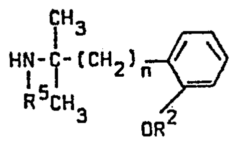

IV

dans laquelle n est 1, 2 ou 3, $R^2$ est l'hydrogène; un groupe alkyle aliphatique contenant 1 à 4 atomes de carbone; le groupe benzyle; un groupe acyle aliphatique contenant 2 à 5 atomes de carbone, ou un groupe benzoyle de formule:

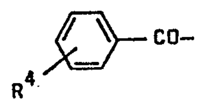

dans laquelle $R^4$ est l'hydrogène ou le groupe méthyle et $R^5$ est l'hydrogène ou un groupe protecteur de l'azote pour former le composé de formule:

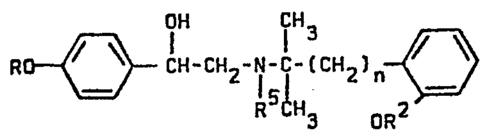

V

après quoi, si nécessaire, les groupes protecteurs R et $R^5$ sont éliminés;

B) à réduire un composé de formule:

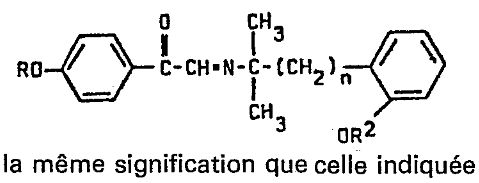

VI

dans laquelle n, R et $R^2$ ont la même signification que celle indiquée sous A ci-dessus pour former un composé de formule:

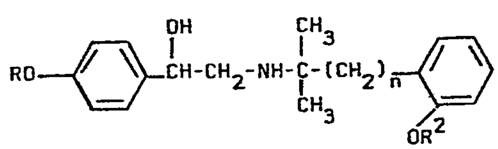

V

après quoi, si nécessaire, le groupe protecteur R est éliminé; et si on le désire à transformer le composé de formule I ainsi obtenu en un sel pharmaceutiquement acceptable et/ou à le séparer en ses isomères optiques.

13. Composition pharmaceutique comprenant comme ingrédient actif un composé répondant à la formule:

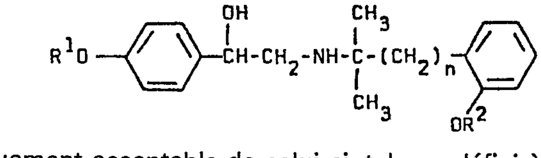

I

ou un sel thérapeutiquement acceptable de celui-ci, tel que défini à la revendication 1, en association avec un véhicule pharmaceutiquement acceptable.

14. Composition pharmaceutique selon la revendication 13, sous la forme de doses unitaires.

15. Composé selon l'une quelconque des revendications 1 à 11, dans son utilisation pour produire la bronchodilatation chez les mammifères, y compris l'homme.

16. Composé selon l'une quelconque des revendications 1 à 11, dans son utilisation pour produire un blocage des $\alpha$-récepteurs chez les mammifères, y compris l'homme.

17. Composé selon l'une quelconque des revendications 1 à 11, dans son utilisation pour produire la relaxation de l'utérus humain.

**Patentansprüche**

1. Äthanolaminverbindungen der Formel

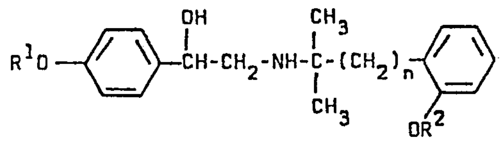

I

und therapeutisch verträgliche Salze derselben, worin n 1, 2 oder 3 ist, $R^1$ ein Wasserstoffatom, eine aliphatische Acylgruppe mit 2 bis 5 Kohlenstoffatomen oder einen Benzoylrest der Formel

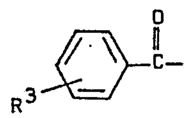

worin $R^3$ ein Wasserstoffatom oder eine methylgruppe ist, bedeutet und worin $R^2$ ein Wasserstoffatom, eine aliphatische Alkylgruppe mit 1 bis 4 Kohlenstoffatomen, die Benzylgruppe, eine aliphatische Acylgruppe mit 2 bis 5 Kohlenstoffatomen oder einen Benzoylrest der Formel

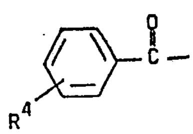

worin $R^4$ ein Wasserstoffatom oder eine Methylgruppe ist, bedeutet.

2. Verbindungen nach Anspruch 1 und therapeutisch verträgliche Salze derselben, worin $R^1$ ein Wasserstoffatom bedeutet.

3. Verbindungen nach Anspruch 1 und therapeutisch verträgliche Salze derselben, worin $R^1$ eine aliphatische Acylgruppe mit 2 bis 5 Kohlenstoffatomen oder einen Benzoyl rest der Formel

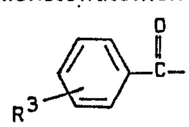

worin $R^3$ ein Wasserstoffatom oder eine Methylgruppe ist, bedeutet.

4. Verbindungen nach Anspruch 1 und therapeutisch verträgliche Salze derselben, worin n 1 ist.

5. Verbindungen nach Anspruch 1 und therapeutisch verträgliche Salze derselben, worin n 2 ist.

6. Verbindungen nach Anspruch 1 und therapeutisch verträgliche Salze derselben, worin $R^2$ eine Alkylgruppe mit 1 bis 4 Kohlenstoffatomen ist.

7. Verbindungen nach Anspruch 1 und therapeutisch verträgliche Salze derselben, worin n 2, $R^1$ ein Wasserstoffatom und $R^2$ eine Alkylgruppe mit 1 bis 4 Kohlenstoffatomen ist.

8. Verbindungen nach Anspruch 1 und therapeutisch verträgliche Salze derselben, worin n 2, $R^1$ eine aliphatische Acylgruppe mit 2 bis 5 Kohlenstoffatomen und $R^2$ eine Alkylgruppe mit 1 bis 4 Kohlenstoffatomen ist.

9. Eine Verbindung der Formel

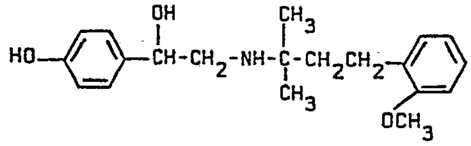

und therapeutisch verträgliche Salze derselben, der Formel

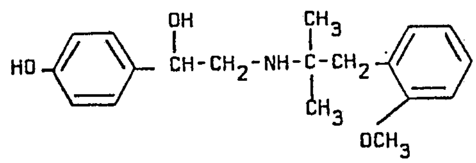

und therapeutisch verträgliche Salze derselben, der Formel

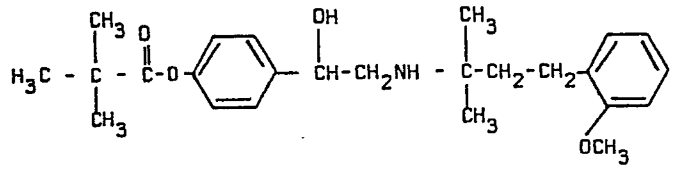

und therapeutisch verträgliche Salze derselben oder der Formel

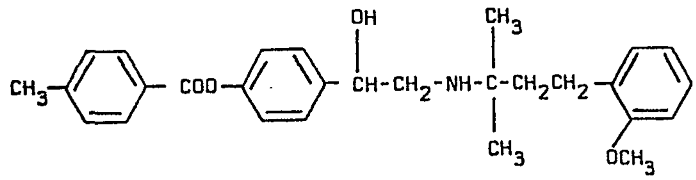

und therapeutisch verträgliche Salze derselben.

10. Eine Verbindung der Formel

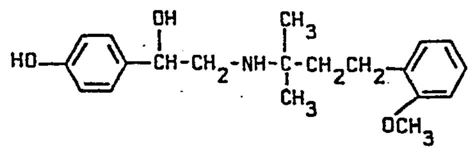

und therapeutisch verträgliche Salze derselben.

11. Eine Verbindung nach einem der Ansprüche 1 bis 10 in der Form
a) eines racemischen Gemisches oder
b) eines im wesentlichen reinen Stereoisomers.

12. Verfahren zur Herstellung von Verbindungen der Formel

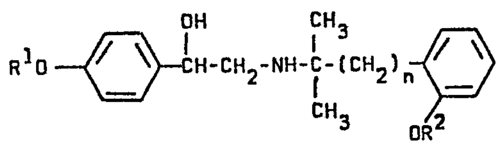

I

und therapeutisch verträglicher Salze derselben, worin n 1, 2 oder 3 bedeutet, $R^1$ ein Wasserstoffatom, eine aliphatische Acylgruppe mit 2 bis 5 Kohlenstoffatomen oder einen Benzoylrest der Formel

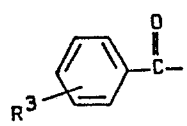

worin $R^3$ ein Wasserstoffatom oder eine Methylgruppe ist, und worin $R^2$ ein Wasserstoffatom, eine aliphatische Alkylgruppe mit 1 bis 4 Kohlenstoffatomen, den Benzylrest, eine aliphatische Acylgruppe mit 2 bis 5 Kohlenstoffatomen oder einen Benzoylrest der Formel

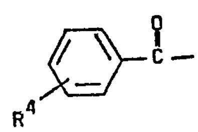

worin $R^4$ ein Wasserstoffatom oder eine Methylgruppe ist, bedeutet, durch

A. Umsetzung einer Verbindung der Formel

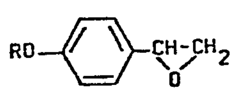

III

worin R ein Wasserstoffatom, eine aliphatische Acylgruppe mit 2 bis 5 Kohlenstoffatomen, ein Benzoylrest der Formel

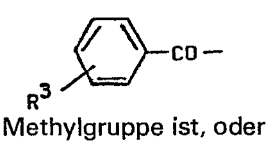

worin $R^3$ ein Wasserstoffatom oder eine Methylgruppe ist, oder ein Hydroxy-Schutzgruppe ist, mit einer Verbindung der Formel

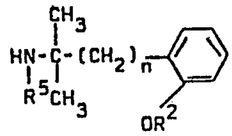

IV

worin n 1, 2 oder 3 ist, $R^2$ ein Wasserstoffatom, eine aliphatische Alkylgruppe mit 1 bis 4 Kohlenstoffatom, der benzylrest, eine aliphatische Acylgruppe mit 2 bis 5 Kohlenstoffatomen oder ein benzoylrest der Formel

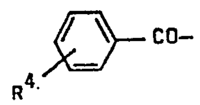

worin $R^4$ ein Wasserstoffatom oder eine Methylgruppe ist, und worin $R^5$ ein Wasserstoffatom oder eine N-Schutzgruppe ist, unter Bildung einer Verbindung der Formel

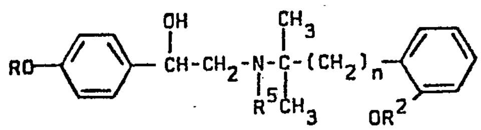

V

wonach, wenn erforderlich, die Schutzgruppen R und $R^5$ entfernt werden, oder
B. Reduktion einer Verbindung der Formel

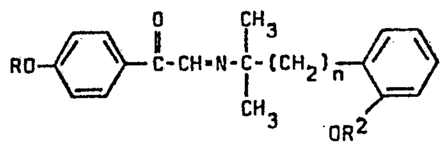

VI

worin n, R and $R^2$ die unter A, oben angegebene Bedeutung haben, unter Bildung einer Verbindung der Formel

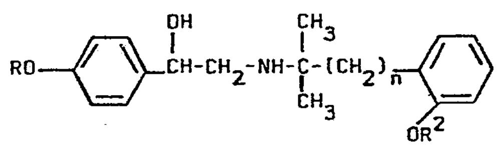

V

wonach, wenn erforderlich, die Schutzgruppe R entfernt wird, wonach, wenn erwünscht, die so erhaltene Verbindung der Formel I in ein pharmazeutisch verträgliches Salz umgewandelt und/oder in ihre optischen Isomeren aufgetrennt wird.

13. Pharmazeutisches Präparat, enthaltend als Wirkstoff eine Verbindung der Formel

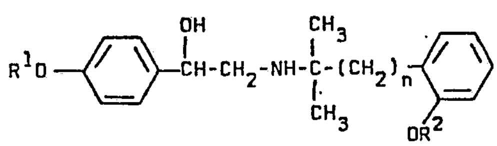

I

oder ein therapeutisch verträgliches Salz derselben gemäß der Definition des Anspruches 1 in Verbindung mit einem pharmazeutisch verträglichen Trägermaterial.

14. Pharmazeutisches Präparat nach Anspruch 13 in der Form von Dosierungseinheiten.

15. Eine Verbindung nach einem der Ansprüche 1 bis 11 für die Verwendung bei der Erzeugung von Bronchienerweiterung bei Säugetieren einschließlich Menschen.

16. Eine Verbindung nach einem der Ansprüche 1 bis 11 für die Verwendung zur Blockierung der $\alpha$-Rezeptoren bei Säugetieren einschließlich Menschen.

17. Eine Verbindung nach einem der Ansprüche 1 bis 11 für die Verwendung bei der Entspannung des menschlischen Uterus.